# EUROPEAN PATENT SPECIFICATION

(11) **EP 1 476 577 B1**
(45) Date of publication and mention of the grant of the patent: **15.04.2009**
(21) Application number: 03733833.2
(22) Date of filing: 21.02.2003
(51) Int. Cl.: C12Q 1/68, C12N 15/85

(54) **REGULATED VECTORS FOR CONTROLLING DNA HYPERMUTABILITY IN EUKARYOTIC CELLS**
REGULIERTE VEKTOREN ZUR STEUERUNG DER DNA-HYPERMUTABILITÄT IN EUKARYONTISCHEN ZELLEN
VECTEURS REGULANT L'HYPERMUTABILITE D'ADN DANS DES CELLULES EUCARYOTES

(30) Priority: 21.02.2002 US 358602 P
(43) Date of publication of application: 17.11.2004
(73) Proprietor: Morphotek, Inc., Exton, PA 19341 (US)
(72) Inventor: GRASSO, Luigi, Pennsylvania , PA 19010 (US); NICOLAIDES, Nicholas, C., Pennsylvania , PA 19061 (US); SASS, Philip, M., Audubon, PA 19403 (US)
(74) Representative: W.P. Thompson & Co.
(86) International application number: PCT/US2003/005193
(87) International publication number: WO 2003/074655

(56) References cited:
- WO-A-01/61012
- WO-A-01/62945
- US-A- 5 464 764
- US-A- 6 146 894
- NICOLAIDES N.C. ET AL.: 'A naturally occurring hPMS2 mutation can confer a dominant negative mutator phenotype' MOLECULAR AND CELLULAR BIOLOGY vol. 18, no. 3, March 1998, pages 1635 - 1641, XP002937919

## Description

### CROSS-REFERENCE TO RELATED APPLICATIONS

### FIELD OF THE INVENTION

The present invention relates to expression vectors containing nucleic acid sequences encoding for a mutator gene linked to a negative selection marker that can be used to positively and negatively regulate DNA hypermutability in eukaryotic cells.

### BACKGROUND OF THE INVENTION

The use of expressing mutator genes, such as but not limited to dominant negative inhibitors of mismatch repair, is a method for generating genetically altered cells to produce subtypes with desired phenotypes. The ability to restore genomic stability via suppressing the expressed mutator allele is critical for stabilizing the genetic integrity of the host's genome. While several methods for regulating gene expression from ectopic vectors exist, the ability to "completely" suppress expression of a mutator transgene is problematic, especially when complete suppression is required to ensure genetic stability. Here, we describe a eukaryotic expression vector system that is capable of producing robust expression levels of a given allele that can be regulated via negative selection to ensure complete suppression.

WO01/62945 discloses a method for making a hypermutable yeast by introducing a dominant negative allele of a mismatch repair gene into the yeast.

US6,146,894 discloses the use of dominant negative alleles of human mismatch repair genes to generate hypermutable cells.

WO01/61012 disclosed the use of dominant negative alleles of mismatch repair genes to generate hypermutable plants.

US5,464,764 disclosed the use of a negative selection marker for selecting a modified target gene.

### SUMMARY OF THE INTENTION

It is the object of the present invention to teach the use of a vector system capable of complete suppression of a mutator transgene within eukaryotic cells.

The invention described herein is the development of a eukaryotic expression vector system for the use in generating genetically evolved cells in a controllable manner. This system entails a constitutively active promoter upstream of a cloning site containing a polylinker suitable for cloning a mutator gene of interest followed by an Internal Ribosome Entry Site and a second polylinker containing a negative selection marker and a polyadenylation site. Internal Ribosome Entry Sites (IRES) are regulatory elements that are found in a number of viruses and cellular RNAs (reviewed in McBratney *et al*. (1993) *Current Opinion in Cell Biology* 5:961). IRES are useful in enhancing translation of a second gene product in a linked eukaryotic expression cassette (Kaufman R.J. *et al*. (1991) *Nucl*. *Acids Res.* 19:4485). This system allows for the constitutive expression of a fusion transcript consisting of an effector mutator gene cDNA followed by a cDNA encoding for a negative selection marker that can be used to select for subclones within a population of cells to identify those that have naturally lost expression.

For the purposes of example, here we demonstrate the development and application of one such vector plasmid referred to as pIRES-PMS134-TK. The pIRES-PMS134-TK plasmid contains a dominant negative mismatch repair gene allele followed by an IRES signal and a negative selection marker derived from herpes simplex virus thymidine kinase (HSV-TK) gene. This vector when transfected in mammalian cells is capable of blocking the endogenous mismatch repair process, leading to subclones with altered phenotypes. Once a subclone with a desired phenotype is identified, the line is made stable through the treatment of ganciclovir (GCV), a prodrug that is converted into a toxic nucleoside analog in cells expressing the HSV-TK gene [Carrio M, Mazo A, Lopez-Iglesias C, Estivill X, Fillat C. (2001) "Retrovirus-mediated transfer of the herpes simplex virus thymidine kinase and connexin26 genes in pancreatic cells results in variable efficiency on the bystander killing: implications for gene therapy." Int. J. Cancer 94:81-88]. Because the mutator cDNA and the HSV-TK are produced from the same transcript, clones that survive GCV treatment are naturally non-expressing the fusion transcript, and exhibit a mismatch repair proficient activity within the host cell.

The use of fusion transcripts containing a mutator gene such as those described by Wood *et al*., [Wood, R.D., Mitchell, M., Sgouros, J., and Lindahl, T. (2001) "Human DNA Repair" Genes Science 1284-1289] and a negative selection marker such as but not limited to the use of vectors containing HSV-tk for ganciclovir selection or the bacterial purine nucleoside phosphorylase gene for 6-methylpurine selection [Gadi V.K., Alexander S.D., Kudlow J.E., Allan P., Parker W.B., Sorscher E.J. (2000) "In vivo sensitization of ovarian tumors to chemotherapy by expression of E. coli purine nucleoside phosphorylase in a small fraction of cells" Gene Ther. 7:1738-1743] has advantages for recombinant methods employing mutator expression vectors to generate genetically diverse clones with *de novo* phenotypes, which in turn can be selected to identify a subset with restored genomic stability.

The vectors of the invention can be employed with the use of a dominant negative inhibitor of MMR in such those previously described [Nicolaides, N.C. et al. (1998) "A naturally occurring hPMS2 mutation can confer a dominant negative mutator phenotype" Mol. Cell. Biol. 18:1635-1641; U.S. Patent No. 6,146,894 to Nicolaides et al.] as an example for developing new cell lines for discovery and product development. A PMS2 homolog may also be encoded refers to a polypeptide sequence having the consensus sequence of AVKE LVENSLDAGA TN (SEQ ID NO:23). In some embodiments, the PMS2 homologs comprise the polypeptide sequence of LRPNAVKE LVENSLDAGA TNVDLKLKDY GVDLIEVSGN GCGVEEENFE (SEQ ID NO:24). The PMS2 homologs comprise this structural feature and, while not wishing to be bound by any particular theory of operation, it is believed that this structural feature correlates with ATPase activity due to the high homology with known ATPases. The knowledge of this structural feature and correlated function and the representative number of examples provided herein, will allow one of ordinary skill in the art to readily identify which proteins may be used in the methods of the invention.

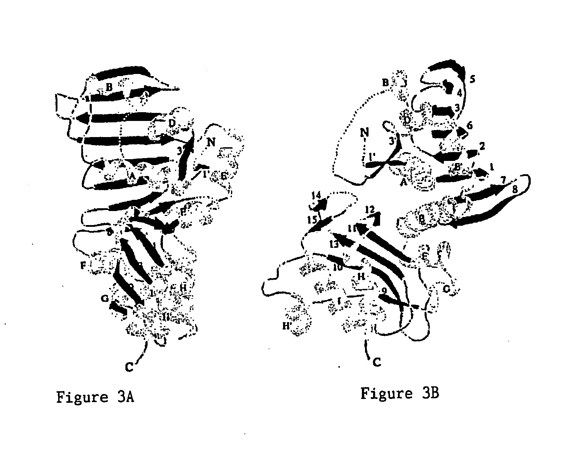

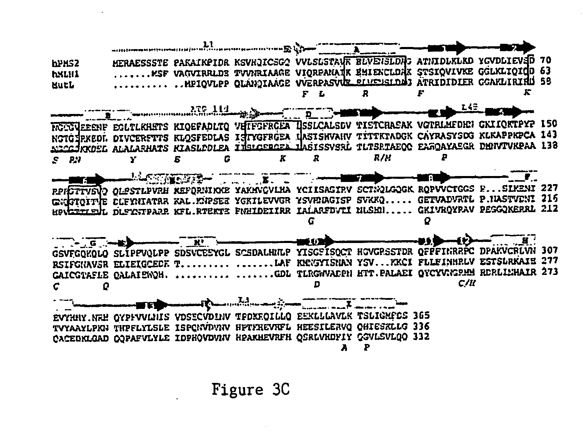

For example, in some embodiments of the invention, the PMS2 homologs may be truncated to lack a C-terminal domain believed to be involved with dimerization. While not wishing to be bound by any particular theory of operation, it is believed that some truncated PMS2 homologs may function by binding ATP as monomers, and mismatch repair activity is decreased due to insufficient heterodimerization between PMS2 and MLH1, however, the conserved N-terminus is intact. Thus, in some embodiments, the PMS2 homologs may further comprise a C-terminal truncation in which the fourth ATP binding motif is eliminated. In some embodiments, the PMS2 homologs comprise a truncation mutation such that the PMS2 homolog is incapable of dimerizing with MLH1. In other embodiments, the PMS2 homologs comprise a truncation from the E' α-helix to the C-terminus (see Figure 3 and Guarne et al. (2001) EMBO J. 20(19):5521-5531). In other embodiments, the PMS2 homologs comprise a truncation from the E α-helix to the C-terminus (see Figure 3 and Guarne et al. (2001) EMBO J. 20(19):5521-5531). In other embodiments, the PMS2 homologs comprise a truncation from the F α-helix to the C-terminus (see Figure 3 and Guarne et al. (2001) EMBO J. 20(19):5521-5531). In other embodiments, the PMS2 homologs comprise a truncation from the G α-helix to the C-terminus (see Figure 3 and Guarne et al. (2001) EMBO J. 20(19):5521-5531). In other embodiments, the PMS2 homologs comprise a truncation from the H' α-helix to the C-terminus (see Figure 3 and Guarne et al. (2001) EMBO J. 20(19):5521-5531). In other embodiments, the PMS2 homologs comprise a truncation from the H α-helix to the C-terminus (see Figure 3 and Guarne et al. (2001) EMBO J. 20(19):5521-5531). In other embodiments, the PMS2 homologs comprise a truncation from the I α-helix to the C-terminus (see Figure 3 and Guarne et al. (2001) EMBO J. 20(19):5521-5531). Any PMS2 homolog that can serve to impart a dominant negative phenotype may be used with the vectors of the invention.

The invention described herein is directed to the creation of selectable expression vectors that can produce mutator proteins that can be suppressed upon negative selection to restore genomic stability.

The present invention describes the development of one such system. The advantages of the present invention are further described in the examples and figures described herein employing dominant negative alleles of MMR linked to the HSV-TK gene as a negative selection marker.

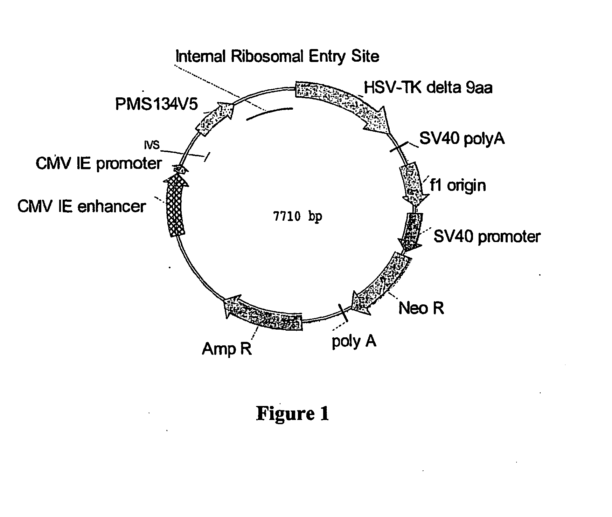

The invention also describes the development and composition of a specific vector that contains the dominant negative MMR gene cDNA PMS134 linked to the HSV-TK negative selection marker as shown in Figure 1.

The invention also provides methods for generating pools and libraries of hypermutable somatic cell lines using methods known by those skilled in the art that can be negatively selected to identify subclones no longer expressing the mutator allele.

The invention also provides methods for generating hypermutable somatic cell lines using methods known by those skilled in the art that can be negatively selected to identify subclones no longer expressing the dominant negative allele, thus exhibiting a genetically stable host.

These and other objects of the invention are provided by one or more of the embodiments described below.

In one embodiment of the invention, a method for making a eukaryotic cell line genetically unstable, followed by selection of a new genotype and reintroduction of a genomic stability is provided. A polynucleotide encoding a dominant negative allele of a MMR gene is introduced into a target cell and the cell line is rendered MMR defective. Pools are generated and selected for subclones with novel phenotypes. Upon confirmation of desired subclones, subclones are expanded and negatively selected for subsets no longer expressing the dominant negative MMK gene allele.

In other embodiments, expression vectors comprising the fusion transcript are able to transform mammalian cells to generate high expression of mutator protein. Thus, another embodiment of the invention is an expression vector comprising a mutator gene fused to IRES sequence followed by a negative selection marker. In a preferred embodiment, the expression vector further comprises a eukaryotic promoter/enhancer driving the expression of a mutator protein of interest. In a most preferred embodiment, the expression vector consists of a fusion plasmid wherein a first gene encodes the gene of interest and a second gene encodes a negative selectable marker. Negative selectable markers include, but not limited to the herpes simplex virus thymidine kinase gene (HSV-TK) or derivatives thereof; other negative selectable markers are also suitable for use in the inventive expression vectors. The fusion expression vector may further comprise an IRES sequence between the two genes. Mammalian cells can be transformed with the expression vectors of the invention, and will produce recombinant mutator protein in a short period of time. Accordingly, another embodiment of the invention provides a mammalian host cell transformed with an expression vector of the invention. In a most preferred embodiment, the host cells are mammalian cells derived of rodent or human origin.

The invention also provides a method for obtaining a cell line with a new phenotype and a stable genome, comprising transforming a host cell with an inventive expression vector, culturing the transformed host cell under conditions promoting expression of the mutator protein, and selecting for new phenotypes. In a preferred application of this invention, transformed host cell lines are selected with two selection steps, the first to select for cells with a new phenotype, and the second step for negative selection of the marker gene to isolate subclones of the cell line exhibiting the desired phenotype that no longer express the mutator protein, thus having restored genomic stability. In a most preferred embodiment, the selection agent is ganciclovir, a prodrug that has been shown to cause toxicity to cells expressing the HSV-TK gene.

In some embodiments, cells may be cultured with a mutagen to increase the frequency of genetic mutations in the cells. The mutagen may be withdrawn upon identification and selection of cells displaying the desired altered phenotype.

These and other embodiments of the invention provide the art with methods that can regulate the genomic stability of a mammalian cell line using genes encoding for mutator proteins linked to a second gene that is useful for negatively selecting clones that no longer express the mutator gene allele.

### BRIEF DESCRIPTION OF THE DRAWINGS

**Figure 1** shows a schematic diagram of a regulated mutator expression plasmid.

**Figure 2** shows the expression of the mutator plasmid before and after negative selection.

**Figure 3A** **and B** show the structure of the N-terminal fragment of PMS2 (orthagonal views) and **Figure 3C** shows a sequence alignment of hPMS2, hMLH1 and MutL (SEQ ID No: 27) N-terminal fragments and structural features corresponding to **Figures 3A** and **B** (from Guarne et al. (2001) EMBO J. 20(19):5521-5531, figure 2 A-C).

### DETAILED DESCRIPTION OF THE INVENTION

The invention provides novel expression vectors that can regulate the DNA stability of a mammalian host cell. One such vector was generated by cloning a mutator gene, which for purposes of this application, employed the dominant negative mismatch repair protein (PMS134) into a fusion transcription vector containing the negative selection marker HSV-TK. The vector of the invention appears to encode a novel function, since the expression of the encoded mutator protein can generate genetically diverse pools of cells that can be selected for novel phenotypes. Once a subline exhibiting a novel phenotype is derived, it can be expanded and selected for subclones that no longer express the mutator gene via a selection using a fused negative selection marker.

### Characterization of selectable mutator fusion genes:

Mutator expression vectors containing a PMS134 (SEQ ID NO:2) cDNA fused to an IRES sequence followed by a negative selection marker gene consisting of a modified HSV-TK gene (SEQ ID NO:1) were generated that are capable of producing proteins from each of these genes (referred to as PMS134-TK). The pIRES-PMS134-TK expression vector (Figure 1) containing this gene was introduced into HEK293 cells. Robust expression of each gene could be detected in HEK293 cells when the fusion transcript was cloned into an expression cassette under control of the cytomegalovirus (CMV) promoter, followed by the SV40 polyadenylation signal. In addition, the vector contained a neomycin selectable marker (NEO) that allowed for positive selection of transfected cells containing the PMS 134-TK gene. The vector of the invention facilitates the regulated expression of a recombinant mutator protein driven by a promoter/enhancer region to which it is linked through the use of negative selection.

Moreover, additional negative selection markers can be used to regulate mutator genes expression in mammalian cells as described herein, as can other mutator gene from other types of cells or from variants of mammalian derived homologs be used such as those described by Woods *et al*. (2001). In addition, it is known in the art that other genes involved in DNA repair can cause a mutator phenotype. Thus, the DNA fragments described and claimed herein also include fragments that encode for such genes can be used.

Other types of fusion fragments described herein can also be developed, for example, fusion genes comprising a mutator gene and a negative selection marker, formed, for example, by juxtaposing the mutator gene open reading frame (ORF) in-frame with the negative selection marker, or positioning the negative selection marker first, followed by the mutator gene. Such combinations can be contiguously linked or arranged to provide optimal spacing for expressing the two gene products (*i*.*e*., by the introduction of "spacer" nucleotides between the gene ORFs). Regulatory elements can also be arranged to provide optimal spacing for expression of both genes such as the use of an IRES motif.

The vector disclosed herein was engineered using the cDNA comprised of the human PMS134 (SEQ ID NO: 2) mutator allele and a modified HSV-TK gene (SEQ ID NO:1). Modified changes to the HSV-TK were made by polymerase chain reaction using the nucleotide sequence set forth in sense primer (5'-ttttctagaccatggcgtctgcgttcg-3' SEQ ID NO:7) which consists of a restriction site to facilitate cloning and a Kozak consensus sequence for efficient translation; and the antisense primer ( 5'-tttgcggccgctacgtgtttcagttagcctcc-3' SEQ ID NO:8) by site-directed mutagenesis techniques that are known in the art.

The expression of recombinant proteins is driven by an appropriate eukaryotic promoter/enhancer and the inventive fusion transcript. Cells are transfected with a plasmid selected under low stringency for the dominant selectable marker and then screened for mutator gene expression. Mutator positive cells are expanded to enhance mutagenesis and selection for a desired phenotype.

Inclusion of an IRES sequence into fusion vectors may be beneficial for enhancing expression of some proteins. The IRES sequence appears to stabilize expression of the genes under selective pressure (Kaufman *et al*. 1991). For some polypeptides, the IRES sequence is not necessary to achieve high expression levels of the downstream sequence.

### Expression Vectors:

Recombinant expression vectors include synthetic or cDNA-derived DNA fragments encoding a mutator protein and a dominant negative selection marker, operably linked to suitable regulatory elements derived from mammalian, viral, or insect genes. Such regulatory elements include a transcriptional promoter, sequences encoding suitable mRNA ribosomal binding sites, and sequences, which control the termination of transcription and translation. Mammalian expression vectors may also comprise nontranscribed elements such as an origin of replication, a suitable promoter and enhancer linked to the gene to be expressed, other 5' or 3' flanking nontranscribed sequences, 5' or 3' nontranslated sequences such as necessary ribosome binding sites, a polyadenylation site, splice donor and acceptor sites, and transcriptional termination sequences. An origin of replication that confers the ability to replicate in a host, and a selectable gene to facilitate recognition of transformants, may also be incorporated. In addition, the expression vector consists of a positive selectable marker that allows for selection of recipient hosts that have taken up the expression vector.

The transcriptional and translational control sequences in expression vectors to be used in transforming vertebrate cells may be provided by viral sources. For example, commonly used promoters and enhancers are derived from human CMV, Adenovirus 2, Simian Virus 40 (SV40), and Polyoma. Viral genomic promoters, control and/or signal sequences may be utilized to drive expression which are dependent upon compatible host cells. Exemplary vectors can be constructed such as described by Okayama and Berg ((1983) Mol. Cell. Biol. 3:280). Promoters derived from house keeping genes can also be used (i.e., the β-globin and the EF-1α promoters), depending on the cell type in which the vector is to be expressed as described (Klehr *et al*. (1991); Grosveld, *et al*., (1987)).

Expression vectors containing internal ribosome entry sites (IRES) used for the expression of multiple transcripts have been described previously (Kim S.K. and B.J. Wold (1985) Cell 42:129; Kaufman *et al*. (1991); Mosley et al. (1989) Cell; Subramani et al., (1981) Mol. Cell. Biol. 1:854). Other IRES-based vectors include those such as the pCDE vector, which contains the IRES derived from the murine encephalomyocarditis virus (Jang and Wimmer, (1990) Genes and Dev. 4:1560), which is cloned between the adenovirus tripartite leader and a DHFR cDNA. Other types of expression vectors will also be useful for regulated expression of mutator genes through negative selection such as those described in U.S. Patent Nos. 4,634,665 to Axel et al. and 4,656,134 to Ringold et al.

Sequences of the modified HSV-TK gene and examples of mutator genes that can be used for enhancing genetic hypermutability include the following: hPMS2-134 (human cDNA) (SEQ ID NO:2); hPMS2-134 (human protein) (SEQ ID NO:12); PMS2 (human protein) (SEQ ID NO:3); PMS2 (human cDNA) (SEQ ID NO:13); PMS1 (human protein) (SEQ ID NO:4); PMS1 (human cDNA) (SEQ ID NO:14); MSH2 (human protein) (SEQ ID NO:5); MSH2 (human cDNA) (SEQ ID NO:15); MLH1 (human protein) (SEQ ID NO:6); MLH1 (human cDNA) (SEQ ID NO:16); PMSR2 (human cDNA Accession No. U38964) (SEQ ID NO:17); hPMSR2 (human protein, Accession No. U38964) (SEQ ID NO:18); HPMSR3 (human cDNA, Accession No. U38979) (SEQ ID NO:19); hPMSR3 (human protein, Accession No. U38979) (SEQ ID NO:20); MLH3 (human protein, Accession No. AAF23904) (SEQ ID NO:21); MLH3 (human cDNA, Accession No. AF195657) (SEQ ID NO:22).

### Host Cells

Transformed host cells are cells which have been transfected with expression vectors generated by recombinant DNA techniques and which contain sequences encoding the fusion transcript containing the mutator gene and negative selection marker. Various mammalian cell culture systems can be employed to express recombinant protein. Examples of suitable mammalian host cell lines include the COS-7 lines of monkey kidney cells, as described by Gluzman (1981) Cell 23:175), and other cell lines capable of expressing an appropriate vector including but not limited to HEK293 (Nicolaides *et al*. 1998), T98G, CV-1/EBNA, L cells (Holst *et al*. (1988)), C127, 3T3, Chinese hamster ovary (CHO) (Weidle, *et al*. (1988)), HeLa TK-ts13 (Nicolaides *et al*. (1998)), NS1, Sp2/0 myeloma cells and BHK cell lines.

### Transfection and generation of stable lines:

In general, transfection will be carried out using a suspension of cells, or a single cell, but other methods can also be applied as long as a sufficient fraction of the treated cells or tissue incorporates the polynucleotide so as to allow transfected cells to be grown and utilized. Techniques for transfection are well known. Several transformation protocols are known in the art, [see Kaufman, R.J. (1988) Meth. Enzymology 185:537]. The transformation protocol employed will depend on the host cell type and the nature of the gene of interest. The basic requirements of any such protocol are first to introduce DNA encoding the protein of interest into a suitable host cell, and then to identify and isolate host cells which have incorporated the vector DNA in a stable, expressible manner. Available techniques for introducing polynucleotides include but are not limited to electroporation, transduction, cell fusion, the use of calcium chloride, and packaging of the polynucleotide together with lipid for fusion with the cells of interest. If the transfection is stable, such that the selectable marker gene is expressed at a consistent level for many cell generations, then a cell line results.

One common method for transfection into mammalian cells is calcium phosphate precipitation as described by Nicolaides *et al*. (1998). Another method is polyethylene glycol (PEG)-induced fusion of bacterial protoplasts with mammalian cells (Schaffner et al. (1980) Proc. Natl. Acad. Sci. USA 77:2163). Yet another method is electroporation. Electroporation can also be used to introduce DNA directly into the cytoplasm of a host cell, for example, as described by Potter et al. (1988) Proc. Natl. Acad. Sci. USA 81:7161.

Transfection of DNA can also be carries out using polyliposome reagents such as Lipofectin and Lipofectamine (Gibco BRL, Gaithersburg, MD) which form lipid-nucleic acid complexes (or liposomes) which, when applied to cultured cells, facilitate uptake of the nucleic acid into the cells.

Once a cell is transfected, pools are selected to identify cells that have taken up the expression vector. Useful dominant selectable markers include microbially derived antibiotic resistance genes, for example neomycin, kanamycin, tetracycline, hygromycin, and penicillin resistance.

The transfected cells may be selected in a number of ways. For cells in which the vector also contains an antibiotic resistance gene, the cells may be selected for antibiotic resistance, which positively selects for cells containing the vector. In other embodiments, the cells may be allowed to mutate, or may be further treated with a mutagen to enhance the rate of mutation and selected based on the presence of an altered phenotype in a gene of interest. Once a phenotype of interest is achieved, the cells may be negatively selected based on the HSV-TK gene such that a genetically stable cell is obtained containing the altered phenotype in the gene of interest.

For further information on the background of the invention the following references may be consulted.

### References

1) Nicolaides, N.C. et al. (1998) A naturally occurring hPMS2 mutation can confer a dominant negative mutator phenotype. Mol. Cell. Biol. 18:1635-1641.
2) Perucho, M. (1996) Cancer of the microsatellite mutator phenotype. Biol. Chem. 377:675-684.
3) Nicolaides, N.C. et al. (1995) Genomic organization of the human PMS2 gene family. Genomics 30:195-206.
4) Nicolaides, N.C. et al. (1997) Interleukin 9: a candidate gene for asthma. Proc. Natl. Acad. Sci. USA 94:13175-13180.
5) Grasso, L. et al. (1998) Molecular analysis of human interleukin-9 receptor transcripts in peripheral blood mononuclear cells. Identification of a splice variant encoding for a nonfunctional cell surface receptor. J. Biol. Chem. 273:24016-24024.
6) Grosveld, F. et al. (1987) Position-independent, high-level expression of the human β-globin gene in transgenic mice. Cell 51:975-955.
7) Holst, A. et al. (1988) Murine genomic DNA sequences replicating autonomously in mouse L cells. Cell 52:355-365.
8) Kaufman, R. et al. (1991) Improved vectors for stable expression of foreign genes in mammalian cells by use of the untranslated leader sequence from EMC virus. Nucl. Acids Res. 19(16):4485-4490, 1991.
9) Klehr, D. et al. (1991) Scaffold-attached regions from the human interferon β domain can be used to enhance the stable expression of genes under the control of various promoters. Biochemistry 30:1264-1270.
10) Lipkin, S.M., et al. (2000) MLH3: a DNA mismatch repair gene associated with mammalian microsatellite instability. Nat. Genet. 24(1):27-35.
11) McBratney, S. et al. (1993) Internal initiation of translation. Curr. Opin. Cell Biol. 5:961-965.
12) Wegner, M. et al. (1990) Interaction of a protein with a palindromic sequence from murine rDNA increases the occurrence of amplification-dependent transformation in mouse cells. J. Biol. Chem. 265(23):13925-13932.
13) Weidle, U. et al. (1988) Amplified expression constructs for human tissue-type plasminogen activator in Chinese hamster ovary cells: instability in the absence of selective pressure Gene 66:193-203.

### EXAMPLES

### Example 1: Engineering the mutator/negative selection fusion.

To demonstrate the utility of mutator/negative selection marker fusions to regulate genomic stability of a host cell, the pIRES-PMS134-TK vector was constructed. The pCMV vector containing the CMV promoter followed by a multiple polylinker cloning site and an SV40 polyadenylation signal was used as a backbone along with a constitutively expressed neomycin phosphotransferase gene as a dominant selectable marker. The pCMV cassette contained an internal ribosome entry site (IRES) from the encephalomyocarditis virus (ECMV) that was cloned within the polylinker region. The gene encoding the PMS134 dominant negative mismatch repair cDNA, when expressed in an otherwise mismatch repair (MMR) proficient cell, renders these host cells MMR deficient [Nicolaides, N.C. et al. (1998) Mol Cell. Biol. 18:1635-1641, U.S. Patent No. 6,146,894.to Nicolaides et al.] The PMS134 gene was cloned into the *Eco*RI*-Xba*I site located upstream of the IRES sequence. The PMS 134 cDNA was modified to facilitate the cloning of the insert as well as to contain a small sequence tag at the C-terminus of the PMS134 polypeptide. The fragment was engineered by PCR using the sense primer (5'-TAATGAATTCACGACTCACTATAGGG-3', SEQ ID:NO 9) that contained an EcoRI site and the antisense primer (5'-TTTGAATTCTCACTACGTAGAATCGAGACCGAG-3' SEQ ID NO:10) that included sequence encoding for the V5 polypeptide fused in-frame with the PMS134 cDNA after residue 133 followed by two termination codons and an EcoRI restriction site. The PMS 134 plasmid (Nicolaides *et al*., 1998) was used as template. PCR products containing the modified PMS134 was digested with EcoRI and cloned into the EcoRI site upstream of the IRES sequence. A modified HSV-TK gene was inserted downstream of the IRES sequence. HSV-TK was modified using a sense (SEQ ID: NO 7) and antisense primer (SEQ ID:NO 8) primer that resulted in the creation of XbaI-NotI fragments that were generated by PCR, digested and subcloned into the expression cassette. A diagram showing the vector, referred to as pIRES-PMS134-TK is given in Figure 1.

FIGURE 1: Schematic pf pIRES-PMS134-TK. (AmpR) = Ampicillin resistance gene; (NeoR) = neomycin phosphotransferase gene fused at the C-temnunus; (PMS134V5) = PMS134 mismatch repair dominant negative gene with a V5 epitope; (HSV-TK delta9aa)= modified herpes simplex virus thymidine kinase gene; (IVS) = intervening sequence from the rabbit β-globin gene; (f1 origin)= bacterial origin of replication (see **Fig. 1**).

### EXAMPLE 2: Generation of negatively selected mutator subclones

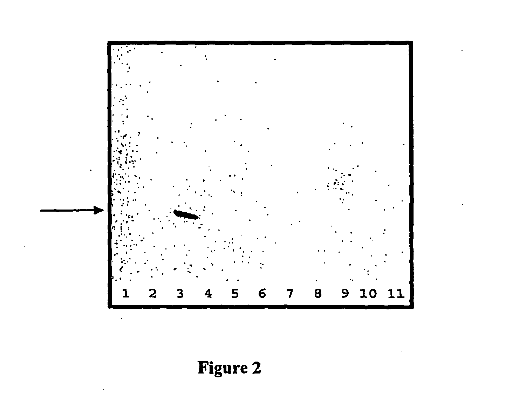

To demonstrate the ability to use a mutator expression vector fused to a negative selection marker as a means to regulate genomic stability of a host cell, the pIRES-PMS 134-TK plasmid was transfected into a MMR proficient human cell line (HEK293). Cells were also transfected with empty vector as a negative control. Cells were transfected using lipofectamine following the manufacturer's recommendations. Transfected pools were selected for 10-14 days in 0.4 mg/ml of G418 (neomycin analog) to select for clones containing the expression vector. Next, cells are analyzed for gene expression via western blot. Briefly, 50,000 cells from the PMS134-TK culture or controls are centrifuged, and resuspended in 150 µls of 2X SDS buffer and cultures were analyzed for PMS 134 protein expression by western blot. Western blots were carried out as follows. 50 µls of each PMS134 or empty vector culture was directly lysed in 2X lysis buffer (60 mM Tris, pH 6.8, 2% SDS, 10% glycerol, 0.1 M 2-mercaptoethanol, 0.001% bromophenol blue) and samples were boiled for 5 minutes. Lysate proteins were separated by electrophoresis on 4-20% Tris glycine gels (Novex). Gels were electroblotted onto Immobilon-P (Millipore) in 48 mM Tris base, 40 mM glycine, 0.0375% SDS, 20% methanol and blocked overnight at 4°C in Tris-buffered saline plus 0.05% Tween-20 and 5% dry milk. Filters were probed with a monoclonal antibody generated against the V5 fusion polypeptide located at the C-terminus of the PMS134 protein, followed by a secondary goat anti-mouse horseradish peroxidase-conjugated antibody. After incubation with the secondary antibody, blots are developed using chemiluminescence (Pierce) and exposed to film to measure PMS134 expression. As shown in **Fig. 2**, a robust expression of PMS134 could be detected in cells containing pIRES-PMS134-TK (**Fig. 2**, lane 3) in contrast to cells expressing empty vector (**Fig. 2**, lane 2), which had no signal.

Next, selected clones expressing PMS 134 are expanded and analyzed for MMR deficiency using methods as previously described. Briefly, cells containing the pIRES-PMS134-TK vector or control were measured for microsatellite instability of endogenous loci using the BAT26 diagnostic marker as described (Hoang J.M. et al. (1997) Cancer Res. 57:300-303). Cells are harvested and genomic DNA is isolated using the salting out method (Nicolaides, N.C. et al. (1991) Mol. Cell. Biol. 11:6166-6176). DNAs are diluted by limiting dilution to determine the level of sensitivity of the assay. DNAs are PCR amplified using the BAT26F: 5'-tgactacttttgacttcagcc-3' (SEQ ID NO: 25)and the BAT26R: 5'-aaccattcaacatttttaaccc-3' (SEQ ID NO: 26) primers in buffers as described (Nicolaides, N.C. et al. (1995) Genomics 30:195-206). Briefly 1 pg to 100 ngs of DNA is amplified using the following conditions: 94°C for 30 sec, 58°C for 30 sec, 72°C for 30 sec for 30 cycles. PCR reactions are electrophoresed on 12% polyacrylamide TBE gels (Novex) or 4% agarose gels and stained with ethidium bromide.

To measure for microsatellite stability in 293 cells, DNAs were amplified using the reaction conditions above. No altered alleles were found in the MMR-proficient control cells as expected since MI only occurs in MMR defective cell hosts, while a number of alterations can be found in PMS134 expressing cells (Perucho, M. (1996)). The results showed that the PMS 134 mutant could exert a robust dominant negative effect, resulting in biochemical and genetic manifestations of MMR deficiency.

Once a hypermutable cell line is developed, it can be screened for to identify subclones with novel phenotypes for target discovery and/or product development. Once the subclone with a desired phenotype is identified, it is important to be able to restore the DNA stability within the host cell. In order to accomplish this, one must remove or completely suppress the expression of the mutator gene. Briefly, cultures are grown in the presence of the prodrug ganciclovir (Sigma), which kills cells expressing the HSV-TK gene product for 5 days. After 5 days, cells are grown for 10 days in growth media alone at which time greater than 95% of cells die off. Resistant clones are then picked and expanded in 10 cm petri dishes. Cells are grown for 3 weeks and then a portion is analyzed for PMS134 expression by western blot and RT-PCR. **Fig. 2** demonstrates a typical result observed in the ganciclovir-resistant cells (GR) whereas robust expression is observed in the untreated PMS134-TK cultures (Figure 2, lane 3), none was observed in the GR resistant cells (Figure 2, lanes 4-11). Analysis of cells after two months of culture have not observed re-expression of the PMS134 gene using western or RT-PCR analysis (not shown). Finally, to demonstrate that genomic stability is restored, cells are analyzed for microsatellite instability as described above. No *de novo* instable microsatellites are observed in the GR cells in contrast to the parental IRES-PMS134-TK line as expected.

Figure 2 shows expression of PMS 134 in negatively selected, ganciclovir resistant HEK293 clones. Wild type (lane 1), empty vector (lane 2), IRES-PMS134-TK pool (lane 3) or IRES-PMS134-TK ganciclovir-resistant selected subclones (lanes 4-11). HEK293 cells were analyzed by western blot for PMS134 expression using an anti-V5 probe that can recognize the PMS134-V5 fusion protein generated by the IRES-PMS134-TK vector as shown in lane 3. Lanes 4-11 are lysates from IRES-PMS134-TK cells negatively selected for expression of the PMS134-HSV-TK fusion transcript after 5 days of ganciclovir exposure. The arrow indicates polypeptide of expected molecular weight.

### Discussion

The results and observation described here lead to several conclusions. First, expression of the PMS134-HSV-TK can generate a mutator phenotype in mammalian cells. Second, pooled cultures of cells containing this expression construct can be negatively selected to restore the genetic stability of the host. These findings teach the use of a vector system suitable for generating stable altered lines for discovery and development using mutator alleles linked to a negative selection marker that can completely inhibit the expression of a mutator expression vector.

## Claims

1. A polynucleotide vector comprising a constitutively active promoter operatively linked to a sequence encoding a dominant negative allele of a mismatch repair gene, an internal ribosome entry site and a negative selection marker sequence.

2. The vector of claim 1 wherein said mismatch repair gene encodes a polypeptide selected from the group consisting of SEQ ID NO:30, SEQ ID NO:4, SEQ ID NO:5, SEQ ID NO:6, SEQ ID NO:12, SEQ ID NO:18, SEQ ID NO:20, and SEQ ID NO:21.

3. The vector of claim 1 or claim 2 wherein said mismatch repair gene comprises a nucleotide sequence selected from the group consisting of SEQ ID NO:2, SEQ ID NO:13, SEQ ID NO:14, SEQ ID NO:15, SEQ ID NO:16, SEQ ID NO:17, SEQ ID NO:19 and SEQ ID NO:22.

4. The vector of claim 1 wherein said dominant negative allele of a mismatch repair gene is PMS134.

5. The vector of any preceding claim further comprising a polyadenylation signal downstream of said selection marker sequence.

6. The vector of any preceding claim wherein said negative selection marker is an HSV-TK gene.

7. The vector of claim 6 wherein said HSV-TK gene comprises the nucleotide sequence of SEQ ID NO:1.

8. The vector of any preceding claim wherein said promoter is selected from the group consisting of a viral promoter including CMV promoter, an adenovirus 2 promoter, an SV40 promoter, and a polyoma promoter.

9. The vector of any of claims 1 to 7 wherein said promoter is selected for host specific expression using constitutively active housekeeping promoters from a host cell's genome.

10. The vector of any preceding claim further comprising a sequence encoding a selectable marker for transfection.

11. The vector of claim 10 wherein said selectable marker for transfection is an antibiotic resistance gene.

12. The vector of claim 11 wherein said antibiotic resistance gene is selected from the group consisting of a neomycin resistance gene, a hygromycin resistance gene, a kanamycin resistance gene, a tetracycline resistance gene, and a penicillin resistance gene.

13. The vector of claim 1 wherein said dominant negative allele of a mismatch repair gene encodes a PMS2 homolog comprising the amino acid sequence of SEQ ID NO:23 or SEQ ID NO:24.

14. A transformed host cell comprising a vector according to any preceding claim.

15. The host cell according to claim 14, wherein said host cell is a eukaryotic cell.

16. A method for producing an isolated, genetically stable cell with a new phenotype, the method comprising the steps of:
a) culturing a recombinant host cell according to claim 14 or claim 15 under conditions for the expression of the dominant negative allele of a mismatch repair gene, rendering the cell hypermutable and thereby producing a library of cells;
b) selecting for clones from the cell library exhibiting new phenotypes whereby positive clones are expanded and propagated; and
c) negatively selecting for clones no longer expressing the dominant negative allele of a mismatch repair gene, rendering the resulting subclones genetically stable.

17. The method of claim 16 wherein said cell is a mammalian cell.

18. The method of claim 16 wherein said cell is a plant cell.

19. The method of claim 16 wherein said cell is an amphibian cell.

20. The method of claim 16 wherein said cell is an insect cell.

21. The method of claim 16 wherein said cell is a fungal cell.

22. The method of any of claims 16 to 21 further comprising treating said cells with a mutagen during said culturing step.

## Patentansprüche

1. Ein Polynucleotidvektor enthaltend im wesentlichen einen aktiven Promoter, der operativ an eine Sequenz gebunden ist, die einen dominant negativen Erbfaktor eines nicht passenden Reparaturgens verschlüsselt, sowie eine innere Ribosomeintrittsstelle und eine negative Auswahlmarkersequenz.

2. Der Vektor nach Anspruch 1, wobei besagtes nicht passendes Reparaturgen ein Polypeptid codiert, welches ausgewählt ist aus der Gruppe, die aus SEQ ID No:3, SEQ ID No:4, SEQ ID No:5, SEQ ID No:6, SEQ ID No:12, SEQ ID No: 18, SEQ ID No:20 und SEQ ID No: 21 besteht.

3. Der Vektor nach Anspruch 1 oder 2, wobei besagtes nicht passendes Reparaturgen eine Nucleotidsequenz enthält, die ausgewählt ist aus der Gruppe, welche aus SEQ ID No:2, SEQ ID No:13, SEQ ID No:14, SEQ ID No:15, SEQ ID No:16, SEQ ID No: 17, SEQ ID No:19 und SEQ ID No: 22 besteht.

4. Der Vektor nach Anspruch 1, wobei besagter dominant negativer Erbgutfaktor des nicht passenden Reparaturgens PMS134 ist.

5. Der Vektor nach einem der vorangegangenen Ansprüche, weiterhin enthaltend ein Polyadenylationssignal stromab besagter Auswahlmarkersequenz.

6. Der Vektor nach einem der vorangegangenen Ansprüche, wobei besagter negativer Auswahlmarker ein HSV-TK Gen ist.

7. Der Vektor nach Anspruch 6, wobei besagtes HSV-TK Gen die Nucleotidsequenz SEQ ID No:1 enthält.

8. Der Vektor nach einem der vorangegangenen Ansprüche, wobei besagter Promoter ausgewählt ist aus der Gruppe, die besteht aus einem viralen Promoter einschließlich CMV Promoter, einem Adenovirus 2 Promoter, einem SV 40 Promoter und einem Polyom Promoter.

9. Der Vektor nach einem der Ansprüche 1 bis 7, wobei besagter Promotor ausgewählt ist zur hostspezifischen Expression unter Verwendung von im wesentlichen aktiver hauswirtschaftlicher Promtoren aus dem Gen einer Hostzelle.

10. Der Vektor nach einem der vorangegangenen Ansprüche weiterhin enthaltend eine Sequenz, die einen auswählbaren Marker zur Transfektion codiert.

11. Der Vektor nach Anspruch 10, wobei besagter auswählbarer Marker zur Transfektion ein antibiotisch resistentes Gen ist.

12. Der Vektor nach Anspruch 11, wobei besagtes antibiotisch resistentes Gen ausgewählt ist aus der Gruppe, die aus einem Neomycin resistenten Gen, einem Hygromycin resistenten Gen, einem Kanamycin resistenten Gen, einem Tretracyclin resistenten Gen und einem Penicillin resistenten Gen besteht.

13. Der Vektor nach Anspruch 1, wobei besagtes dominant negatives Erbgut eines nicht passenden Reparaturgens ein PMS2 Homolog codiert enthaltend die Aminosäuresequenz von SEQ ID No: 23 oder SEQ ID No: 24.

14. Eine transformierte Hostzelle enthaltend einen Vektor nach einem der vorangegangenen Ansprüche.

15. Hostzelle nach Anspruch 14, wobei besagte Zelle eine eukaryotische Zelle ist.

16. Verfahren zur Herstellung einer isolierten genetisch stabilen Zelle mit einem neuen Phenotyp, die Methode enthaltend folgende Schritte:
a) Kultivierung einer rekombinanten Hostzelle nach Anspruch 14 oder 15 unter Bedingungen für die Expression des dominant negativen Erbgutfaktors eines nicht passenden Reparaturgens, diese Zelle hypermutierbar übergebend und auf diese Weise eine Zellbibliothek erstellend:
b) Auswahl von Klonen aus der Zellbibliothek unter Ausstellung neuer Phenotypen, wobei positive Klone expandiert und fortgepflanzt werden sowie
c) negative Auswahl zum Klonen nicht mehr unter Ausdruck der dominant negativen Erbgutfaktoren eines nicht passenden Reparaturgens, auf diese Weise die resultierenden Subklone genetisch stabil erhaltend.

17. Verfahren nach Anspruch 16, wobei besagte Zelle eine mammalische Zelle ist.

18. Verfahren nach Anspruch 16, wobei besagte Zelle eine Pflanzenzelle ist.

19. Verfahren nach Anspruch 16, wobei besagte Zelle eine amphibische Zelle ist.

20. Verfahren nach Anspruch 16, wobei besagte Zelle eine Insektenzelle ist.

21. Verfahren nach Anspruch 16, wobei besagte Zelle eine Pilzzelle ist.

22. Verfahren nach einem der Ansprüche 16 bis 21 weiterhin enthaltend die Behandlung besagter Zellen mit einem Mutagen während besagten Schrittes zur Kultivierung.

## Revendications

1. Vecteur polynucléotidique comprenant un promoteur constitutivement actif lié de manière fonctionnelle à une séquence codant pour un allèle dominant négatif d'un gène de réparation des mésappariements, un site interne d'entrée des ribosomes et une séquence de marqueur de sélection négative.

2. Vecteur selon la revendication 1, dans lequel ledit gène de réparation des mésappariements, code pour un polypeptide choisi dans le groupe constitué de SEQ ID NO : 3, SEQ ID NO : 4, SEQ ID NO : 5, SEQ ID NO : 6, SEQ ID NO : 12, SEQ ID NO : 18, SEQ ID NO : 20 et SEQ ID NO : 21.

3. Vecteur selon à revendication 1 ou la revendication 2, dans lequel ledit gène de réparation des mésappariements comprend une séquence nucléotidique choisie dans le groupe constitué de SEQ ID NO : 2, SEQ ID NO : 13, SEQ ID NO : 14, SEQ ID NO : 15, SEQ ID NO : 16, SEQ ID NO : 17, SEQ ID NO : 19 et SEQ ID NO : 22.

4. Vecteur selon la revendication 1, dans lequel ledit allèle dominant négatif d'un gène de réparation des mésappariements est PMS134.

5. Vecteur selon l'une quelconque des revendications précédentes comprenant en outre un signal de polyadénylation en aval de ladite séquence de marqueur de sélection.

6. Vecteur selon l'une quelconque des revendications précédentes, dans lequel ledit marqueur de sélection négative est un gène HSV-TK.

7. Vecteur selon la revendication 6, dans lequel ledit gène HSV-TK comprend la séquence nucléotidique de SEQ ID NO : 1.

8. Vecteur selon l'une quelconque des revendications précédentes, dans lequel ledit promoteur est choisi dans le groupe constitué d'un promoteur viral y compris le promoteur CMV, un promoteur d'adénovirus 2, un promoteur SV40, un promoteur de polyome.

9. Vecteur selon l'une quelconque des revendications 1 à 7, dans lequel ledit promoteur est choisi pour une expression spécifique de l'hôte en utilisant des promoteurs domestiques constitutivement actifs provenant du génome d'une cellule hôte.

10. Vecteur selon l'une quelconque des revendications précédentes, comprenant en outre une séquence codant pour un marqueur sélectionnable pour une transfection.

11. Vecteur selon la revendication 10, dans lequel ledit marqueur sélectionnable pour une transfection est un gène de résistance à un antibiotique.

12. Vecteur selon la revendication 11, dans lequel ledit gène de résistance à un antibiotique est choisi dans le groupe constitué d'un gène de résistance à à néomycine, d'un gène de résistance à l'hygromycine, d'un gène de résistance à la kanamycine, d'un gène de résistance à la tétracycline et d'un gène de résistance à la pénicilline.

13. Vecteur selon la revendication 1, dans lequel ledit allèle dominant négatif d'un gène de réparation des mésappariements code pour un homologue de PMS2 comprenant la séquence d'acides aminés de SEQ ID NO : 23 ou SEQ ID NO : 24.

14. Cellule hôte transformée comprenant un vecteur selon l'une quelconque des revendications précédentes.

15. Cellule hôte selon la revendication 14, où ladite cellule hôte est une cellule eucaryote.

16. Procédé de production d'une cellule génétiquement stable, isolée avec un nouveau phénotype, le procédé comprenant les étapes consistant à :
a) cultiver une cellule hôte recombinante selon la revendication 14 ou la revendication 15 dans des conditions pour l'expression de l'allèle dominant négatif d'un gène de réparation des mésappariements, rendant la cellule hypermutable et de cette manière produisant une banque de cellules ;
b) sélectionner des clones à partir de la banque de cellules présentant de nouveaux phénotypes, moyennant quoi les clones positifs sont soumis à une expansion et une propagation ; et
c) sélectionner négativement des clones n'exprimant plus l'allèle dominant négatif d'un gène de réparation des mésappariements, rendant les sous-clones résultants génétiquement stables.

17. Procédé selon la revendication 16, dans lequel ladite cellule est une cellule mammalienne.

18. Procédé selon la revendication 16, dans lequel ladite cellule est une cellule végétale.

19. Procédé selon la revendication 16, dans lequel ladite cellule est une cellule d'amphibien.

20. Procédé selon la revendication 16, dans lequel ladite cellule est une cellule d'insecte.

21. Procédé selon la revendication 16, dans lequel ladite cellule est une cellule fongique.

22. Procédé selon l'une quelconque des revendications 16 à 21, comprenant en outre le traitement desdites cellules avec un mutagène au cours de ladite étape de culture.


### SEQUENCE LISTING

<110> Morphotek Inc.
   Grasso, Luigi
   Nicolaides, Nicholas C.
   Sass, Philip M.
<120> REGULATED VECTORS FOR CONTROLLING DNA HYPERMUTABILITY IN EUKARIOTIC CELLS
<130> MOR-0345
<150> PCT/US03/05193
   <151> 2003-02-21
<150> US 60/358,602
   <151> 2002-02-21
<160> 27
<170> PatentIn version 3.3
<210> 1
   <211> 1252
   <212> DNA
   <213> Artificial
<220>
   <223> Vector Sequence
<400> 1
   atggcttcgt acccctgcca tcaacacgcg tctgcgttcg accaggctgc gcgttctcgc 60
   ggccatagca accgacgtac ggcgttgcgc cctcgccggc agcaagaagc cacggaagtc 120
   cgcctggagc agaaaatgcc cacgctactg cgggtttata tagacggtcc tcacgggatg 180
   gggaaaacca ccaccacgca actgctggtg gccctgggtt cgcgcgacga tatcgtctac 240
   gtacccgagc cgatgactta ctggcaggtg ctgggggctt ccgagacaat cgcgaacatc 300
   tacaccacac aacaccgcct cgaccagggt gagatatcgg ccggggacgc ggcggtggta 360
   atgacaagcg cccagataac aatgggcatg ccttatgccg tgaccgacgc cgttctggct 420
   cctcatatcg ggggggaggc tgggagctca catgccccgc ccccggccct caccctcatc 480
   ttcgaccgcc atcccatcgc cgccctcctg tgctacccgg ccgcgcggta ccttatgggc 540
   agcatgaccc cccaggccgt gctggcgttc gtggccctca tcccgccgac cttgcccggc 600
   accaacatcg tgcttggggc ccttccggag gacagacaca tcgaccgcct ggccaaacgc 660
   cagcgccccg gcgagcggct ggacctggct atgctggctg cgattcgccg cgtttacggg
   720
   ctacttgcca atacggtgcg gtatctgcag ggcggcgggt cgtggcggga ggattgggga
   780
   cagctttcgg ggacggccgt gccgccccag ggtgccgagc cccagagcaa cgcgggccca
   840
   cgaccccata tcggggacac gttatttacc ctgtttcggg cccccgagtt gctggccccc
   900
   aacggcgacc tgtataacgt gtttgcctgg gccttggacg tcttggccaa acgcctccgt
   960
   cccatgcacg tctttatcct ggattacgac caatcgcccg ccggctaccg ggacgccctg
   1020
   ctgcaactta cctccgggat ggtccagacc cacgtcacca ccccaggctc cataccgacg
   1080
   atctgcgacc tggcgcgcac gtttgcccgg gagatggggg aggctaactg aaacacggaa
   1140
   ggagacaata ccggaaggaa cccgcgctat gacggcaata aaaagacaga ataaaacgca
   1200
   cgggtgttgg gtcgtttgtt cataaacgcg gggttcggtc ccagggctgg ca
   1252
<210> 2
   <211> 426
   <212> DNA
   <213> Homo sapiens
<400> 2
   cgaggcggat cgggtgttgc atccatggag cgagctgaga gctcgagtac agaacctgct
   60
   aaggccatca aacctattga tcggaagtca gtccatcaga tttgctctgg gcaggtggta
   120
   ctgagtctaa gcactgcggt aaaggagtta gtagaaaaca gtctggatgc tggtgccact
   180
   aatattgatc taaagcttaa ggactatgga gtggatctta ttgaagtttc agacaatgga
   240
   tgtggggtag aagaagaaaa cttcgaaggc ttaactctga aacatcacac atctaagatt
   300
   caagagtttg ccgacctaac tcaggttgaa acttttggct ttcgggggga agctctgagc
   360
   tcactttgtg cactgagcga tgtcaccatt tctacctgcc acgcatcggc gaaggttgga
   420
   acttga
   426
<210> 3
   <211> 862
   <212> PRT
   <213> Homo sapiens
<400> 3
   Met Glu Arg Ala Glu Ser Ser Ser Thr Glu Pro Ala Lys Ala Ile Lys
   1               5                   10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr Arg Leu Met Phe Asp His Asn Gly Lys Ile Ile
       130                 135                 140
   Gln Lys Thr Pro Tyr Pro Arg Pro Arg Gly Thr Thr Val Ser Val Gln
   145                 150                 155                 160
   Gln Leu Phe Ser Thr Leu Pro Val Arg His Lys Glu Phe Gln Arg Asn
                   165                 170                 175
   Ile Lys Lys Glu Tyr Ala Lys Met Val Gln Val Leu His Ala Tyr Cys
               180                 185                 190
   Ile Ile Ser Ala Gly Ile Arg Val Ser Cys Thr Asn Gln Leu Gly Gln
           195                 200                 205
   Gly Lys Arg Gln Pro Val Val Cys Thr Gly Gly Ser Pro Ser Ile Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ser Val Cys Glu Glu Tyr Gly
                   245                 250                 255
   Leu Ser Cys Ser Asp Ala Leu His Asn Leu Phe Tyr Ile Ser Gly Phe
               260                 265                 270
   Ile Ser Gln Cys Thr His Gly Val Gly Arg Ser Ser Thr Asp Arg Gln
           275                 280                 285
   Phe Phe Phe Ile Asn Arg Arg Pro Cys Asp Pro Ala Lys Val Cys Arg
       290                 295                 300
   Leu Val Asn Glu Val Tyr His Met Tyr Asn Arg His Gln Tyr Pro Phe
   305                 310                 315                 320
   Val Val Leu Asn Ile Ser Val Asp Ser Glu Cys Val Asp Ile Asn Val
                   325                 330                 335
   Thr Pro Asp Lys Arg Gln Ile Leu Leu Gln Glu Glu Lys Leu Leu Leu
               340                 345                 350
   Ala Val Leu Lys Thr Ser Leu Ile Gly Met Phe Asp Ser Asp Val Asn
           355                 360                 365
   Lys Leu Asn Val Ser Gln Gln Pro Leu Leu Asp Val Glu Gly Asn Leu
       370                 375                 380
   Ile Lys Met His Ala Ala Asp Leu Glu Lys Pro Met Val Glu Lys Gln
   385                 390                 395                 400
   Asp Gln Ser Pro Ser Leu Arg Thr Gly Glu Glu Lys Lys Asp Val Ser
                   405                 410                 415
   Ile Ser Arg Leu Arg Glu Ala Phe Ser Leu Arg His Thr Thr Glu Asn
               420                 425                 430
   Lys Pro His Ser Pro Lys Thr Pro Glu Pro Arg Arg Ser Pro Leu Gly
           435                 440                 445
   Gln Lys Arg Gly Met Leu Ser Ser Ser Thr Ser Gly Ala Ile Ser Asp
       450                 455                 460
   Lys Gly Val Leu Arg Pro Gln Lys Glu Ala Val Ser Ser Ser His Gly
   465                 470                 475                 480
   Pro Ser Asp Pro Thr Asp Arg Ala Glu Val Glu Lys Asp Ser Gly His
                   485                 490                 495
   Gly Ser Thr Ser Val Asp Ser Glu Gly Phe Ser Ile Pro Asp Thr Gly
               500                 505                 510
   Ser His Cys Ser Ser Glu Tyr Ala Ala Ser Ser Pro Gly Asp Arg Gly
   515 520 525
   Ser Gln Glu His Val Asp Ser Gln Glu Lys Ala Pro Glu Thr Asp Asp
   530 535 540
   Ser Phe Ser Asp Val Asp Cys His Ser Asn Gln Glu Asp Thr Gly Cys
   545 550 555 560
   Lys Phe Arg Val Leu Pro Gln Pro Thr Asn Leu Ala Thr Pro Asn Thr
   565 570 575
   Lys Arg Phe Lys Lys Glu Glu Ile Leu Ser Ser Ser Asp Ile Cys Gln
   580 585 590
   Lys Leu Val Asn Thr Gln Asp Met Ser Ala Ser Gln Val Asp Val Ala
   595 600 605
   Val Lys Ile Asn Lys Lys Val Val Pro Leu Asp Phe Ser Met Ser Ser
   610 615 620
   Leu Ala Lys Arg Ile Lys Gln Leu His His Glu Ala Gln Gln Ser Glu
   625 630 635 640
   Gly Glu Gln Asn Tyr Arg Lys Phe Arg Ala Lys Ile Cys Pro Gly Glu
   645 650 655
   Asn Gln Ala Ala Glu Asp Glu Leu Arg Lys Glu Ile Ser Lys Thr Met
   660 665 670
   Phe Ala Glu Met Glu Ile Ile Gly Gln Phe Asn Leu Gly Phe Ile Ile
   675 680 685
   Thr Lys Leu Asn Glu Asp Ile Phe Ile Val Asp Gln His Ala Thr Asp
   690 695 700
   Glu Lys Tyr Asn Phe Glu Met Leu Gln Gln His Thr Val Leu Gln Gly
   705 710 715 720
   Gln Arg Leu Ile Ala Pro Gln Thr Leu Asn Leu Thr Ala Val Asn Glu
   725 730 735
   Ala Val Leu Ile Glu Asn Leu Glu Ile Phe Arg Lys Asn Gly Phe Asp
   740 745 750
   Phe Val Ile Asp Glu Asn Ala Pro Val Thr Glu Arg Ala Lys Leu Ile
   755 760 765
   Ser Leu Pro Thr Ser Lys Asn Trp Thr Phe Gly Pro Gln Asp Val Asp
       770                 775                 780
   Glu Leu Ile Phe Met Leu Ser Asp Ser Pro Gly Val Met Cys Arg Pro
   785                 790                 795                 800
   Ser Arg Val Lys Gln Met Phe Ala Ser Arg Ala Cys Arg Lys Ser Val
                   805                 810                 815
   Met Ile Gly Thr Ala Leu Asn Thr Ser Glu Met Lys Lys Leu Ile Thr
               820                 825                 830
   His Met Gly Glu Met Asp His Pro Trp Asn Cys Pro His Gly Arg Pro
           835                 840                 845
   Thr Met Arg His Ile Ala Asn Leu Gly Val Ile Ser Gln Asn
       850                 855                 860
<210> 4
   <211> 932
   <212> PRT
   <213> Homo sapiens
<400> 4
<210> 5
   <211> 934
   <212> PRT
   <213> Homo sapiens
<400> 5
<210> 6
   <211> 756
   <212> PRT
   <213> Homo sapiens
<400> 6
<210> 7
   <211> 27
   <212> DNA
   <213> Artificial
<220>
   <223> Oligonucleotide Primer
<400> 7
   ttttctagac catggcgtct gcgttcg
   27
<210> 8
   <211> 32
   <212> DNA
   <213> Artificial
<220>
   <223> Oligonucleotide Primer
<400> 8
   tttgcggccg ctacgtgttt cagttagcct cc
   32
<210> 9
   <211> 26
   <212> DNA
   <213> Artificial
<220>
   <223> Oligonucleotide Primer
<400> 9
   taatgaattc acgactcact ataggg
   26
<210> 10
   <211> 33
   <212> DNA
   <213> Artificial
<220>
   <223> Oligonucleotide Primer
<400> 10
   tttgaattct cactacgtag aatcgagacc gag
   33
<210> 11
   <211> 292
   <212> PRT
   <213> Artificial
<220>
   <223> human PMS2:herpes simplex TK chimera
<220>
   <221> misc_feature
   <222> (5) .. (5)
   <223> Xaa can be any naturally occurring amino acid
<220>
   <221> misc_feature
   <222> (37)..(37)
   <223> Xaa can be any naturally occurring amino acid
<400> 11
   Met Thr Tyr Trp Xaa Val Leu Gly Ala Ser Glu Thr Ile Ala Asn Ile
   1 5 10 15
   Tyr Thr Thr Gln His Arg Leu Asp Gln Gly Glu Ile Ser Ala Gly Asp
   20 25 30
   Ala Ala Val Val Xaa Thr Ser Ala Gln Ile Thr Met Gly Met Pro Tyr
   35 40 45
   Ala Val Thr Asp Ala Val Leu Ala Pro His Ile Gly Gly Glu Ala Gly
   50 55 60
   Ser Ser His Ala Pro Pro Pro Ala Leu Thr Leu Ile Phe Asp Arg His
   65 70 75 80
   Pro Ile Ala Ala Leu Leu Cys Tyr Pro Ala Ala Arg Tyr Leu Met Gly
   85 90 95
   Ser Met Thr Pro Gln Ala Val Leu Ala Phe Val Ala Leu Ile Pro Pro
   100 105 110
   Thr Leu Pro Gly Thr Asn Ile Val Leu Gly Ala Leu Pro Glu Asp Arg
   115 120 125
   His Ile Asp Arg Leu Ala Lys Arg Gln Arg Pro Gly Glu Arg Leu Asp
   130 135 140
   Leu Ala Met Leu Ala Ala Ile Arg Arg Val Tyr Gly Leu Leu Ala Asn
   145 150 155 160
   Thr Val Arg Tyr Leu Gln Cys Gly Gly Ser Trp Arg Glu Asp Trp Gly
   165 170 175
   Gln Leu Ser Gly Thr Ala Val Pro Pro Gln Gly Ala Glu Pro Gln Ser
   180 185 190
   Asn Ala Gly Pro Arg Pro His Ile Gly Asp Thr Leu Phe Thr Leu Phe
   195 200 205
   Arg Ala Pro Glu Leu Leu Ala Pro Asn Gly Asp Leu Tyr Asn Val Phe
   210 215 220
   Ala Trp Ala Leu Asp Val Leu Ala Lys Arg Leu Arg Ser Met His Val
   225 230 235 240
   Phe Ile Leu Asp Tyr Asp Gln Ser Pro Ala Gly Cys Arg Asp Ala Leu
   245 250 255
   Leu Gln Leu Thr Ser Gly Met Val Gln Thr His Val Thr Thr Pro Gly
               260                 265                 270
   Ser Ile Pro Thr Ile Cys Asp Leu Ala Arg Thr Phe Ala Arg Glu Met
           275                 280                 285
   Gly Glu Ala Asn
       290
<210> 12
   <211> 133
   <212> PRT
   <213> Homo sapiens
<400> 12
   Met Glu Arg Ala Glu Ser Ser Ser Thr Glu Pro Ala Lys Ala Ile Lys
   1               5                   10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr
       130
<210> 13
   <211> 2771
   <212> DNA
   <213> Homo sapiens
<400> 13
   cgaggcggat cgggtgttgc atccatggag cgagctgaga gctcgagtac agaacctgct
   60
   aaggccatca aacctattga tcggaagtca gtccatcaga tttgctctgg gcaggtggta 120
   ctgagtctaa gcactgcggt aaaggagtta gtagaaaaca gtctggatgc tggtgccact 180
   aatattgatc taaagcttaa ggactatgga gtggatctta ttgaagtttc agacaatgga 240
   tgtggggtag aagaagaaaa cttcgaaggc ttaactctga aacatcacac atctaagatt 300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   actcgactga tgtttgatca caatgggaaa attatccaga aaacccccta cccccgcccc 480
   agagggacca cagtcagcgt gcagcagtta ttttccacac tacctgtgcg ccataaggaa 540
   tttcaaagga atattaagaa ggagtatgcc aaaatggtcc aggtcttaca tgcatactgt 600
   atcatttcag caggcatccg tgtaagttgc accaatcagc ttggacaagg aaaacgacag 660
   cctgtggtat gcacaggtgg aagccccagc ataaaggaaa atatcggctc tgtgtttggg 720
   cagaagcagt tgcaaagcct cattcctttt gttcagctgc cccctagtga ctccgtgtgt 780
   gaagagtacg gtttgagctg ttcggatgct ctgcataatc ttttttacat ctcaggtttc 840
   atttcacaat gcacgcatgg agttggaagg agttcaacag acagacagtt tttctttatc 900
   aaccggcggc cttgtgaccc agcaaaggtc tgcagactcg tgaatgaggt ctaccacatg 960
   tataatcgac accagtatcc atttgttgtt cttaacattt ctgttgattc agaatgcgtt 1020
   gatatcaatg ttactccaga taaaaggcaa attttgctac aagaggaaaa gcttttgttg 1080
   gcagttttaa agacctcttt gataggaatg tttgatagtg atgtcaacaa gctaaatgtc 1140
   agtcagcagc cactgctgga tgttgaaggt aacttaataa aaatgcatgc agcggatttg 1200
   gaaaagccca tggtagaaaa gcaggatcaa tccccttcat taaggactgg agaagaaaaa 1260
   aaagacgtgt ccatttccag actgcgagag gccttttctc ttcgtcacac aacagagaac 1320
   aagcctcaca gcccaaagac tccagaacca agaaggagcc ctctaggaca gaaaaggggt 1380
   atgctgtctt ctagcacttc aggtgccatc tctgacaaag gcgtcctgag acctcagaaa
   1440
   gaggcagtga gttccagtca cggacccagt gaccctacgg acagagcgga ggtggagaag
   1500
   gactcggggc acggcagcac ttccgtggat tctgaggggt tcagcatccc agacacgggc
   1560
   agtcactgca gcagcgagta tgcggccagc tccccagggg acaggggctc gcaggaacat
   1620
   gtggactctc aggagaaagc gcctgaaact gacgactctt tttcagatgt ggactgccat
   1680
   tcaaaccagg aagataccgg atgtaaattt cgagttttgc ctcagccaac taatctcgca
   1740
   accccaaaca caaagcgttt taaaaaagaa gaaattcttt ccagttctga catttgtcaa
   1800
   aagttagtaa atactcagga catgtcagcc tctcaggttg atgtagctgt gaaaattaat
   1860
   aagaaagttg tgcccctgga cttttctatg agttctttag ctaaacgaat aaagcagtta
   1920
   catcatgaag cacagcaaag tgaaggggaa cagaattaca ggaagtttag ggcaaagatt
   1980
   tgtcctggag aaaatcaagc agccgaagat gaactaagaa aagagataag taaaacgatg
   2040
   tttgcagaaa tggaaatcat tggtcagttt aacctgggat ttataataac caaactgaat
   2100
   gaggatatct tcatagtgga ccagcatgcc acggacgaga agtataactt cgagatgctg
   2160
   cagcagcaca ccgtgctcca ggggcagagg ctcatagcac ctcagactct caacttaact
   2220
   gctgttaatg aagctgttct gatagaaaat ctggaaatat ttagaaagaa tggctttgat
   2280
   tttgttatcg atgaaaatgc tccagtcact gaaagggcta aactgatttc cttgccaact
   2340
   agtaaaaact ggaccttcgg accccaggac gtcgatgaac tgatcttcat gctgagcgac
   2400
   agccctgggg tcatgtgccg gccttcccga gtcaagcaga tgtttgcctc cagagcctgc
   2460
   cggaagtcgg tgatgattgg gactgctctt aacacaagcg agatgaagaa actgatcacc
   2520
   cacatggggg agatggacca cccctggaac tgtccccatg gaaggccaac catgagacac
   2580
   atcgccaacc tgggtgtcat ttctcagaac tgaccgtagt cactgtatgg aataattggt
   2640
   tttatcgcag atttttatgt tttgaaagac agagtcttca ctaacctttt ttgttttaaa
   2700
   atgaaacctg ctacttaaaa aaaatacaca tcacacccat ttaaaagtga tcttgagaac
   2760
   cttttcaaac c
   2771
<210> 14
   <211> 3063
   <212> DNA
   <213> Homo sapiens
<400> 14
   ggcacgagtg gctgcttgcg gctagtggat ggtaattgcc tgcctcgcgc tagcagcaag
   60
   ctgctctgtt aaaagcgaaa atgaaacaat tgcctgcggc aacagttcga ctcctttcaa
   120
   gttctcagat catcacttcg gtggtcagtg ttgtaaaaga gcttattgaa aactccttgg
   180
   atgctggtgc cacaagcgta gatgttaaac tggagaacta tggatttgat aaaattgagg
   240
   tgcgagataa cggggagggt atcaaggctg ttgatgcacc tgtaatggca atgaagtact
   300
   acacctcaaa aataaatagt catgaagatc ttgaaaattt gacaacttac ggttttcgtg
   360
   gagaagcctt ggggtcaatt tgttgtatag ctgaggtttt aattacaaca agaacggctg
   420
   ctgataattt tagcacccag tatgttttag atggcagtgg ccacatactt tctcagaaac
   480
   cttcacatct tggtcaaggt acaactgtaa ctgctttaag attatttaag aatctacctg
   540
   taagaaagca gttttactca actgcaaaaa aatgtaaaga tgaaataaaa aagatccaag
   600
   atctcctcat gagctttggt atccttaaac ctgacttaag gattgtcttt gtacataaca
   660
   aggcagttat ttggcagaaa agcagagtat cagatcacaa gatggctctc atgtcagttc
   720
   tggggactgc tgttatgaac aatatggaat cctttcagta ccactctgaa gaatctcaga
   780
   tttatctcag tggatttctt ccaaagtgtg atgcagacca ctctttcact agtctttcaa
   840
   caccagaaag aagtttcatc ttcataaaca gtcgaccagt acatcaaaaa gatatcttaa
   900
   agttaatccg acatcattac aatctgaaat gcctaaagga atctactcgt ttgtatcctg
   960
   ttttctttct gaaaatcgat gttcctacag ctgatgttga tgtaaattta acaccagata
   1020
   aaagccaagt attattacaa aataaggaat ctgttttaat tgctcttgaa aatctgatga
   1080
   cgacttgtta tggaccatta cctagtacaa attcttatga aaataataaa acagatgttt
   1140
   ccgcagctga catcgttctt agtaaaacag cagaaacaga tgtgcttttt aataaagtgg
   1200
   aatcatctgg aaagaattat tcaaatgttg atacttcagt cattccattc caaaatgata
   1260
   tgcataatga tgaatctgga aaaaacactg atgattgttt aaatcaccag ataagtattg
   1320
   gtgactttgg ttatggtcat tgtagtagtg aaatttctaa cattgataaa aacactaaga
   1380
   atgcatttca ggacatttca atgagtaatg tatcatggga gaactctcag acggaatata
   1440
   gtaaaacttg ttttataagt tccgttaagc acacccagtc agaaaatggc aataaagacc
   1500
   atatagatga gagtggggaa aatgaggaag aagcaggtct tgaaaactct tcggaaattt
   1560
   ctgcagatga gtggagcagg ggaaatatac ttaaaaattc agtgggagag aatattgaac
   1620
   ctgtgaaaat tttagtgcct gaaaaaagtt taccatgtaa agtaagtaat aataattatc
   1680
   caatccctga acaaatgaat cttaatgaag attcatgtaa caaaaaatca aatgtaatag
   1740
   ataataaatc tggaaaagtt acagcttatg atttacttag caatcgagta atcaagaaac
   1800
   ccatgtcagc aagtgctctt tttgttcaag atcatcgtcc tcagtttctc atagaaaatc
   1860
   ctaagactag tttagaggat gcaacactac aaattgaaga actgtggaag acattgagtg
   1920
   aagaggaaaa actgaaatat gaagagaagg ctactaaaga cttggaacga tacaatagtc
   1980
   aaatgaagag agccattgaa caggagtcac aaatgtcact aaaagatggc agaaaaaaga
   2040
   taaaacccac cagcgcatgg aatttggccc agaagcacaa gttaaaaacc tcattatcta
   2100
   atcaaccaaa acttgatgaa ctccttcagt cccaaattga aaaaagaagg agtcaaaata
   2160
   ttaaaatggt acagatcccc ttttctatga aaaacttaaa aataaatttt aagaaacaaa
   2220
   acaaagttga cttagaagag aaggatgaac cttgcttgat ccacaatctc aggtttcctg
   2280
   atgcatggct aatgacatcc aaaacagagg taatgttatt aaatccatat agagtagaag
   2340
   aagccctgct atttaaaaga cttcttgaga atcataaact tcctgcagag ccactggaaa
   2400
   agccaattat gttaacagag agtcttttta atggatctca ttatttagac gttttatata
   2460
   aaatgacagc agatgaccaa agatacagtg gatcaactta cctgtctgat cctcgtctta
   2520
   cagcgaatgg tttcaagata aaattgatac caggagtttc aattactgaa aattacttgg
   2580
   aaatagaagg aatggctaat tgtctcccat tctatggagt agcagattta aaagaaattc
   2640
   ttaatgctat attaaacaga aatgcaaagg aagtttatga atgtagacct cgcaaagtga
   2700
   taagttattt agagggagaa gcagtgcgtc tatccagaca attacccatg tacttatcaa
   2760
   aagaggacat ccaagacatt atctacagaa tgaagcacca gtttggaaat gaaattaaag
   2820
   agtgtgttca tggtcgccca ttttttcatc atttaaccta tcttccagaa actacatgat
   2880
   taaatatgtt taagaagatt agttaccatt gaaattggtt ctgtcataaa acagcatgag
   2940
   tctggtttta aattatcttt gtattatgtg tcacatggtt attttttaaa tgaggattca
   3000
   ctgacttgtt tttatattga aaaaagttcc acgtattgta gaaaacgtaa ataaactaat
   3060
   aac
   3063
<210> 15
   <211> 3145
   <212> DNA
   <213> Homo sapiens
<400> 15
   ggcgggaaac agcttagtgg gtgtggggtc gcgcattttc ttcaaccagg aggtgaggag 60
   gtttcgacat ggcggtgcag ccgaaggaga cgctgcagtt ggagagcgcg gccgaggtcg 120
   gcttcgtgcg cttctttcag ggcatgccgg agaagccgac caccacagtg cgccttttcg 180
   accggggcga cttctatacg gcgcacggcg aggacgcgct gctggccgcc cgggaggtgt 240
   tcaagaccca gggggtgatc aagtacatgg ggccggcagg agcaaagaat ctgcagagtg 300
   ttgtgcttag taaaatgaat tttgaatctt ttgtaaaaga tcttcttctg gttcgtcagt 360
   atagagttga agtttataag aatagagctg gaaataaggc atccaaggag aatgattggt
   420
   atttggcata taaggcttct cctggcaatc tctctcagtt tgaagacatt ctctttggta
   480
   acaatgatat gtcagcttcc attggtgttg tgggtgttaa aatgtccgca gttgatggcc
   540
   agagacaggt tggagttggg tatgtggatt ccatacagag gaaactagga ctgtgtgaat
   600
   tccctgataa tgatcagttc tccaatcttg aggctctcct catccagatt ggaccaaagg
   660
   aatgtgtttt acccggagga gagactgctg gagacatggg gaaactgaga cagataattc
   720
   aaagaggagg aattctgatc acagaaagaa aaaaagctga cttttccaca aaagacattt
   780
   atcaggacct caaccggttg ttgaaaggca aaaagggaga gcagatgaat agtgctgtat
   840
   tgccagaaat ggagaatcag gttgcagttt catcactgtc tgcggtaatc aagtttttag
   900
   aactcttatc agatgattcc aactttggac agtttgaact gactactttt gacttcagcc
   960
   agtatatgaa attggatatt gcagcagtca gagcccttaa cctttttcag ggttctgttg
   1020
   aagataccac tggctctcag tctctggctg ccttgctgaa taagtgtaaa acccctcaag
   1080
   gacaaagact tgttaaccag tggattaagc agcctctcat ggataagaac agaatagagg
   1140
   agagattgaa tttagtggaa gcttttgtag aagatgcaga attgaggcag actttacaag
   1200
   aagatttact tcgtcgattc ccagatctta accgacttgc caagaagttt caaagacaag
   1260
   cagcaaactt acaagattgt taccgactct atcagggtat aaatcaacta cctaatgtta
   1320
   tacaggctct ggaaaaacat gaaggaaaac accagaaatt attgttggca gtttttgtga
   1380
   ctcctcttac tgatcttcgt tctgacttct ccaagtttca ggaaatgata gaaacaactt
   1440
   tagatatgga tcaggtggaa aaccatgaat tccttgtaaa accttcattt gatcctaatc
   1500
   tcagtgaatt aagagaaata atgaatgact tggaaaagaa gatgcagtca acattaataa
   1560
   gtgcagccag agatcttggc ttggaccctg gcaaacagat taaactggat tccagtgcac
   1620
   agtttggata ttactttcgt gtaacctgta aggaagaaaa agtccttcgt aacaataaaa
   1680
   actttagtac tgtagatatc cagaagaatg gtgttaaatt taccaacagc aaattgactt
   1740
   ctttaaatga agagtatacc aaaaataaaa cagaatatga agaagcccag gatgccattg
   1800
   ttaaagaaat tgtcaatatt tcttcaggct atgtagaacc aatgcagaca ctcaatgatg
   1860
   tgttagctca gctagatgct gttgtcagct ttgctcacgt gtcaaatgga gcacctgttc
   1920
   catatgtacg accagccatt ttggagaaag gacaaggaag aattatatta aaagcatcca
   1980
   ggcatgcttg tgttgaagtt caagatgaaa ttgcatttat tcctaatgac gtatactttg
   2040
   aaaaagataa acagatgttc cacatcatta ctggccccaa tatgggaggt aaatcaacat
   2100
   atattcgaca aactggggtg atagtactca tggcccaaat tgggtgtttt gtgccatgtg
   2160
   agtcagcaga agtgtccatt gtggactgca tcttagcccg agtaggggct ggtgacagtc
   2220
   aattgaaagg agtctccacg ttcatggctg aaatgttgga aactgcttct atcctcaggt
   2280
   ctgcaaccaa agattcatta ataatcatag atgaattggg aagaggaact tctacctacg
   2340
   atggatttgg gttagcatgg gctatatcag aatacattgc aacaaagatt ggtgcttttt
   2400
   gcatgtttgc aacccatttt catgaactta ctgccttggc caatcagata ccaactgtta
   2460
   ataatctaca tgtcacagca ctcaccactg aagagacctt aactatgctt tatcaggtga
   2520
   agaaaggtgt ctgtgatcaa agttttggga ttcatgttgc agagcttgct aatttcccta
   2580
   agcatgtaat agagtgtgct aaacagaaag ccctggaact tgaggagttt cagtatattg
   2640
   gagaatcgca aggatatgat atcatggaac cagcagcaaa gaagtgctat ctggaaagag
   2700
   agcaaggtga aaaaattatt caggagttcc tgtccaaggt gaaacaaatg ccctttactg
   2760
   aaatgtcaga agaaaacatc acaataaagt taaaacagct aaaagctgaa gtaatagcaa
   2820
   agaataatag ctttgtaaat gaaatcattt cacgaataaa agttactacg tgaaaaatcc
   2880
   cagtaatgga atgaaggtaa tattgataag ctattgtctg taatagtttt atattgtttt
   2940
   atattaaccc tttttccata gtgttaactg tcagtgccca tgggctatca acttaataag
   3000
   atatttagta atattttact ttgaggacat tttcaaagat ttttattttg aaaaatgaga
   3060
   gctgtaactg aggactgttt gcaattgaca taggcaataa taagtgatgt gctgaatttt
   3120
   ataaataaaa tcatgtagtt tgtgg
   3145
<210> 16
   <211> 2271
   <212> DNA
   <213> Homo sapiens
<400> 16
   atgtcgttcg tggcaggggt tattcggcgg ctggacgaga cagtggtgaa ccgcatcgcg
   60
   gcgggggaag ttatccagcg gccagctaat gctatcaaag agatgattga gaactgttta
   120
   gatgcaaaat ccacaagtat tcaagtgatt gttaaagagg gaggcctgaa gttgattcag
   180
   atccaagaca atggcaccgg gatcaggaaa gaagatctgg atattgtatg tgaaaggttc
   240
   actactagta aactgcagtc ctttgaggat ttagccagta tttctaccta tggctttcga
   300
   ggtgaggctt tggccagcat aagccatgtg gctcatgtta ctattacaac gaaaacagct
   360
   gatggaaagt gtgcatacag agcaagttac tcagatggaa aactgaaagc ccctcctaaa
   420
   ccatgtgctg gcaatcaagg gacccagatc acggtggagg acctttttta caacatagcc
   480
   acgaggagaa aagctttaaa aaatccaagt gaagaatatg ggaaaatttt ggaagttgtt
   540
   ggcaggtatt cagtacacaa tgcaggcatt agtttctcag ttaaaaaaca aggagagaca
   600
   gtagctgatg ttaggacact acccaatgcc tcaaccgtgg acaatattcg ctccatcttt
   660
   ggaaatgctg ttagtcgaga actgatagaa attggatgtg aggataaaac cctagccttc
   720
   aaaatgaatg gttacatatc caatgcaaac tactcagtga agaagtgcat cttcttactc
   780
   ttcatcaacc atcgtctggt agaatcaact tccttgagaa aagccataga aacagtgtat
   840
   gcagcctatt tgcccaaaaa cacacaccca ttcctgtacc tcagtttaga aatcagtccc
   900
   cagaatgtgg atgttaatgt gcaccccaca aagcatgaag ttcacttcct gcacgaggag
   960
   agcatcctgg agcgggtgca gcagcacatc gagagcaagc tcctgggctc caattcctcc
   1020
   aggatgtact tcacccagac tttgctacca ggacttgctg gcccctctgg ggagatggtt
   1080
   aaatccacaa caagtctgac ctcgtcttct acttctggaa gtagtgataa ggtctatgcc
   1140
   caccagatgg ttcgtacaga ttcccgggaa cagaagcttg atgcatttct gcagcctctg
   1200
   agcaaacccc tgtccagtca gccccaggcc attgtcacag aggataagac agatatttct
   1260
   agtggcaggg ctaggcagca agatgaggag atgcttgaac tcccagcccc tgctgaagtg
   1320
   gctgccaaaa atcagagctt ggagggggat acaacaaagg ggacttcaga aatgtcagag
   1380
   aagagaggac ctacttccag caaccccaga aagagacatc gggaagattc tgatgtggaa
   1440
   atggtggaag atgattcccg aaaggaaatg actgcagctt gtaccccccg gagaaggatc
   1500
   attaacctca ctagtgtttt gagtctccag gaagaaatta atgagcaggg acatgaggtt
   1560
   ctccgggaga tgttgcataa ccactccttc gtgggctgtg tgaatcctca gtgggccttg
   1620
   gcacagcatc aaaccaagtt ataccttctc aacaccacca agcttagtga agaactgttc
   1680
   taccagatac tcatttatga ttttgccaat tttggtgttc tcaggttatc ggagccagca
   1740
   ccgctctttg accttgccat gcttgcctta gatagtccag agagtggctg gacagaggaa
   1800
   gatggtccca aagaaggact tgctgaatac attgttgagt ttctgaagaa gaaggctgag
   1860
   atgcttgcag actatttctc tttggaaatt gatgaggaag ggaacctgat tggattaccc
   1920
   cttctgattg acaactatgt gccccctttg gagggactgc ctatcttcat tcttcgacta
   1980
   gccactgagg tgaattggga cgaagaaaag gaatgttttg aaagcctcag taaagaatgc
   2040
   gctatgttct attccatccg gaagcagtac atatctgagg agtcgaccct ctcaggccag
   2100
   cagagtgaag tgcctggctc cattccaaac tcctggaagt ggactgtgga acacattgtc
   2160
   tataaagcct tgcgctcaca cattctgcct cctaaacatt tcacagaaga tggaaatatc
   2220
   ctgcagcttg ctaacctgcc tgatctatac aaagtctttg agaggtgtta a
   2271
<210> 17
   <211> 1408
   <212> DNA
   <213> Homo sapiens
<400> 17
   ggcgctccta cctgcaagtg gctagtgcca agtgctgggc cgccgctcct gccgtgcatg 60
   ttggggagcc agtacatgca ggtgggctcc acacggagag gggcgcagac ccggtgacag 120
   ggctttacct ggtacatcgg catggcgcaa ccaaagcaag agagggtggc gcgtgccaga 180
   caccaacggt cggaaaccgc cagacaccaa cggtcggaaa ccgccaagac accaacgctc 240
   ggaaaccgcc agacaccaac gctcggaaac cgccagacac caaggctcgg aatccacgcc 300
   aggccacgac ggagggcgac tacctccctt ctgaccctgc tgctggcgtt cggaaaaaac 360
   gcagtccggt gtgctctgat tggtccaggc tctttgacgt cacggactcg acctttgaca 420
   gagccactag gcgaaaagga gagacgggaa gtattttttc cgccccgccc ggaaagggtg 480
   gagcacaacg tcgaaagcag ccgttgggag cccaggaggc ggggcgcctg tgggagccgt 540
   ggagggaact ttcccagtcc ccgaggcgga tccggtgttg catccttgga gcgagctgag 600
   aactcgagta cagaacctgc taaggccatc aaacctattg atcggaagtc agtccatcag 660
   atttgctctg ggccggtggt accgagtcta aggccgaatg cggtgaagga gttagtagaa 720
   aacagtctgg atgctggtgc cactaatgtt gatctaaagc ttaaggacta tggagtggat 780
   ctcattgaag tttcaggcaa tggatgtggg gtagaagaag aaaacttcga aggctttact 840
   ctgaaacatc acacatgtaa gattcaagag tttgccgacc taactcaggt ggaaactttt 900
   ggctttcggg gggaagctct gagctcactt tgtgcactga gtgatgtcac catttctacc 960
   tgccgtgtat cagcgaaggt tgggactcga ctggtgtttg atcactatgg gaaaatcatc 1020
   cagaaaaccc cctacccccg ccccagaggg atgacagtca gcgtgaagca gttattttct 1080
   acgctacctg tgcaccataa agaatttcaa aggaatatta agaagaaacg tgcctgcttc
   1140
   cccttcgcct tctgccgtga ttgtcagttt cctgaggcct ccccagccat gcttcctgta
   1200
   cagcctgtag aactgactcc tagaagtacc ccaccccacc cctgctcctt ggaggacaac
   1260
   gtgatcactg tattcagctc tgtcaagaat ggtccaggtt cttctagatg atctgcacaa
   1320
   atggttcctc tcctccttcc tgatgtctgc cattagcatt ggaataaagt tcctgctgaa
   1380
   aatccaaaaa aaaaaaaaaa aaaaaaaa
   1408
<210> 18
   <211> 389
   <212> PRT
   <213> Homo sapiens
<400> 18
   Met Ala Gln Pro Lys Gln Glu Arg Val Ala Arg Ala Arg His Gln Arg
   1               5                   10                  15
   Ser Glu Thr Ala Arg His Gln Arg Ser Glu Thr Ala Lys Thr Pro Thr
               20                  25                  30
   Leu Gly Asn Arg Gln Thr Pro Thr Leu Gly Asn Arg Gln Thr Pro Arg
           35                  40                  45
   Leu Gly Ile His Ala Arg Pro Arg Arg Arg Ala Thr Thr Ser Leu Leu
       50                  55                  60
   Thr Leu Leu Leu Ala Phe Gly Lys Asn Ala Val Arg Cys Ala Leu Ile
   65                  70                  75                  80
   Gly Pro Gly Ser Leu Thr Ser Arg Thr Arg Pro Leu Thr Glu Pro Leu
                   85                  90                  95
   Gly Glu Lys Glu Arg Arg Glu Val Phe Phe Pro Pro Arg Pro Glu Arg
               100                 105                 110
   Val Glu His Asn Val Glu Ser Ser Arg Trp Glu Pro Arg Arg Arg Gly
           115                 120                 125
   Ala Cys Gly Ser Arg Gly Gly Asn Phe Pro Ser Pro Arg Gly Gly Ser
       130                 135                 140
   Gly Val Ala Ser Leu Glu Arg Ala Glu Asn Ser Ser Thr Glu Pro Ala
   145                 150                 155                 160
   Lys Ala Ile Lys Pro Ile Asp Arg Lys Ser Val His Gln Ile Cys Ser
                   165                 170                 175
   Gly Pro Val Val Pro Ser Leu Arg Pro Asn Ala Val Lys Glu Leu Val
               180                 185                 190
   Glu Asn Ser Leu Asp Ala Gly Ala Thr Asn Val Asp Leu Lys Leu Lys
           195                 200                 205
   Asp Tyr Gly Val Asp Leu Ile Glu Val Ser Gly Asn Gly Cys Gly Val
       210                 215                 220
   Glu Glu Glu Asn Phe Glu Gly Phe Thr Leu Lys His His Thr Cys Lys
   225                 230                 235                 240
   Ile Gln Glu Phe Ala Asp Leu Thr Gln Val Glu Thr Phe Gly Phe Arg
                   245                 250                 255
   Gly Glu Ala Leu Ser Ser Leu Cys Ala Leu Ser Asp Val Thr Ile Ser
               260                 265                 270
   Thr Cys Arg Val Ser Ala Lys Val Gly Thr Arg Leu Val Phe Asp His
           275                 280                 285
   Tyr Gly Lys Ile Ile Gln Lys Thr Pro Tyr Pro Arg Pro Arg Gly Met
       290                 295                 300
   Thr Val Ser Val Lys Gln Leu Phe Ser Thr Leu Pro Val His His Lys
   305                 310                 315                 320
   Glu Phe Gln Arg Asn Ile Lys Lys Lys Arg Ala Cys Phe Pro Phe Ala
                   325                 330                 335
   Phe Cys Arg Asp Cys Gln Phe Pro Glu Ala Ser Pro Ala Met Leu Pro
               340                 345                 350
   Val Gln Pro Val Glu Leu Thr Pro Arg Ser Thr Pro Pro His Pro Cys
           355                 360                 365
   Ser Leu Glu Asp Asn Val Ile Thr Val Phe Ser Ser Val Lys Asn Gly
       370                 375                 380
   Pro Gly Ser Ser Arg
   385
<210> 19
   <211> 1785
   <212> DNA
   <213> Homo sapiens
<400> 19
   tttttagaaa ctgatgttta ttttccatca accatttttc catgctgctt aagagaatat 60
   gcaagaacag cttaagacca gtcagtggtt gctcctaccc attcagtggc ctgagcagtg 120
   gggagctgca gaccagtctt ccgtggcagg ctgagcgctc cagtcttcag tagggaattg 180
   ctgaataggc acagagggca cctgtacacc ttcagaccag tctgcaacct caggctgagt 240
   agcagtgaac tcaggagcgg gagcagtcca ttcaccctga aattcctcct tggtcactgc 300
   cttctcagca gcagcctgct cttctttttc aatctcttca ggatctctgt agaagtacag 360
   atcaggcatg acctcccatg ggtgttcacg ggaaatggtg ccacgcatgc gcagaacttc 420
   ccgagccagc atccaccaca ttaaacccac tgagtgagct cccttgttgt tgcatgggat 480
   ggcaatgtcc acatagcgca gaggagaatc tgtgttacac agcgcaatgg taggtaggtt 540
   aacataagat gcctccgtga gaggcgaagg ggcggcggga cccgggcctg gcccgtatgt 600
   gtccttggcg gcctagacta ggccgtcgct gtatggtgag ccccagggag gcggatctgg 660
   gcccccagaa ggacacccgc ctggatttgc cccgtagccc ggcccgggcc cctcgggagc 720
   agaacagcct tggtgaggtg gacaggaggg gacctcgcga gcagacgcgc gcgccagcga 780
   cagcagcccc gccccggcct ctcgggagcc gggggcagga ggctgcggag ccccaggagg 840
   gtctatcagc cacagtctct gcatgtttcc aagagcaaca ggaaatgaac acattgcagg 900
   ggccagtgtc attcaaagat gtggctgtgg atttcaccca ggaggagtgg cggcaactgg 960
   accctgatga gaagatagca tacggggatg tgatgttgga gaactacagc catctagttt 1020
   ctgtggggta tgattatcac caagccaaac atcatcatgg agtggaggtg aaggaagtgg 1080
   agcagggaga ggagccgtgg ataatggaag gtgaatttcc atgtcaacat agtccagaac 1140
   ctgctaaggc catcaaacct attgatcgga agtcagtcca tcagatttgc tctgggccag 1200
   tggtactgag tctaagcact gcagtgaagg agttagtaga aaacagtctg gatgctggtg 1260
   ccactaatat tgatctaaag cttaaggact atggagtgga tctcattgaa gtttcagaca
   1320
   atggatgtgg ggtagaagaa gaaaactttg aaggcttaat ctctttcagc tctgaaacat
   1380
   cacacatgta agattcaaga gtttgccgac ctaactgaag ttgaaacttt cggttttcag
   1440
   ggggaagctc tgagctcact gtgtgcactg agcgatgtca ccatttctac ctgccacgcg
   1500
   ttggtgaagg ttgggactcg actggtgttt gatcacgatg ggaaaatcat ccaggaaacc
   1560
   ccctaccccc accccagagg gaccacagtc agcgtgaagc agttattttc tacgctacct
   1620
   gtgcgccata aggaatttca aaggaatatt aagaagacgt gcctgcttcc ccttcgcctt
   1680
   ctgccgtgat tgtcagtttc ctgaggcctc cccagccatg cttcctgtac agcctgcaga
   1740
   actgtgagtc aattaaacct cttttcttca taaattaaaa aaaaa
   1785
<210> 20
   <211> 264
   <212> PRT
   <213> Homo sapiens
<400> 20
   Met Cys Pro Trp Arg Pro Arg Leu Gly Arg Arg Cys Met Val Ser Pro
   1 5 10 15
   Arg Glu Ala Asp Leu Gly Pro Gln Lys Asp Thr Arg Leu Asp Leu Pro
   20 25 30
   Arg Ser Pro Ala Arg Ala Pro Arg Glu Gln Asn Ser Leu Gly Glu Val
   35 40 45
   Asp Arg Arg Gly Pro Arg Glu Gln Thr Arg Ala Pro Ala Thr Ala Ala
   50 55 60
   Pro Pro Arg Pro Leu Gly Ser Arg Gly Ala Glu Ala Ala Glu Pro Gln
   65 70 75 80
   Glu Gly Leu Ser Ala Thr Val Ser Ala Cys Phe Gln Glu Gln Gln Glu
   85 90 95
   Met Asn Thr Leu Gln Gly Pro Val Ser Phe Lys Asp Val Ala Val Asp
   100 105 110
   Phe Thr Gln Glu Glu Trp Arg Gln Leu Asp Pro Asp Glu Lys Ile Ala
   115 120 125
   Tyr Gly Asp Val Met Leu Glu Asn Tyr Ser His Leu Val Ser Val Gly
   130 135 140
   Tyr Asp Tyr His Gln Ala Lys His His His Gly Val Glu Val Lys Glu
   145 150 155 160
   Val Glu Gln Gly Glu Glu Pro Trp Ile Met Glu Gly Glu Phe Pro Cys
   165 170 175
   Gln His Ser Pro Glu Pro Ala Lys Ala Ile Lys Pro Ile Asp Arg Lys
   180 185 190
   Ser Val His Gln Ile Cys Ser Gly Pro Val Val Leu Ser Leu Ser Thr
   195 200 205
   Ala Val Lys Glu Leu Val Glu Asn Ser Leu Asp Ala Gly Ala Thr Asn
   210 215 220
   Ile Asp Leu Lys Leu Lys Asp Tyr Gly Val Asp Leu Ile Glu Val Ser
   225 230 235 240
   Asp Asn Gly Cys Gly Val Glu Glu Glu Asn Phe Glu Gly Leu Ile Ser
   245 250 255
   Phe Ser Ser Glu Thr Ser His Met
   260
<210> 21
   <211> 1429
   <212> PRT
   <213> Homo sapiens
<400> 21
   Met Ile Lys Cys Leu Ser Val Glu Val Gln Ala Lys Leu Arg Ser Gly
   1               5                   10                  15
   Leu Ala Ile Ser Ser Leu Gly Gln Cys Val Glu Glu Leu Ala Leu Asn
               20                  25                  30
   Ser Ile Asp Ala Glu Ala Lys Cys Val Ala Val Arg Val Asn Met Glu
           35                  40                  45
   Thr Phe Gln Val Gln Val Ile Asp Asn Gly Phe Gly Met Gly Ser Asp
       50                  55                  60
   Asp Val Glu Lys Val Gly Asn Arg Tyr Phe Thr Ser Lys Cys His Ser
   65                  70                  75                  80
   Val Gln Asp Leu Glu Asn Pro Arg Phe Tyr Gly Phe Arg Gly Glu Ala
   85 90 95
   Leu Ala Asn Ile Ala Asp Met Ala Ser Ala Val Glu Ile Ser Ser Lys
   100 105 110
   Lys Asn Arg Thr Met Lys Thr Phe Val Lys Leu Phe Gln Ser Gly Lys
   115 120 125
   Ala Leu Lys Ala Cys Glu Ala Asp Val Thr Arg Ala Ser Ala Gly Thr
   130 135 140
   Thr Val Thr Val Tyr Asn Leu Phe Tyr Gln Leu Pro Val Arg Arg Lys
   145 150 155 160
   Cys Met Asp Pro Arg Leu Glu Phe Glu Lys Val Arg Gln Arg Ile Glu
   165 170 175
   Ala Leu Ser Leu Met His Pro Ser Ile Ser Phe Ser Leu Arg Asn Asp
   180 185 190
   Val Ser Gly Ser Met Val Leu Gln Leu Pro Lys Thr Lys Asp Val Cys
   195 200 205
   Ser Arg Phe Cys Gln Ile Tyr Gly Leu Gly Lys Ser Gln Lys Leu Arg
   210 215 220
   Glu Ile Ser Phe Lys Tyr Lys Glu Phe Glu Leu Ser Gly Tyr Ile Ser
   225 230 235 240
   Ser Glu Ala His Tyr Asn Lys Asn Met Gln Phe Leu Phe Val Asn Lys
   245 250 255
   Arg Leu Val Leu Arg Thr Lys Leu His Lys Leu Ile Asp Phe Leu Leu
   260 265 270
   Arg Lys Glu Ser Ile Ile Cys Lys Pro Lys Asn Gly Pro Thr Ser Arg
   275 280 285
   Gln Met Asn Ser Ser Leu Arg His Arg Ser Thr Pro Glu Leu Tyr Gly
   290 295 300
   Ile Tyr Val Ile Asn Val Gln Cys Gln Phe Cys Glu Tyr Asp Val Cys
   305 310 315 320
   Met Glu Pro Ala Lys Thr Leu Ile Glu Phe Gln Asn Trp Asp Thr Leu
   325 330 335
   Leu Phe Cys Ile Gln Glu Gly Val Lys Met Phe Leu Lys Gln Glu Lys
   340 345 350
   Leu Phe Val Glu Leu Ser Gly Glu Asp Ile Lys Glu Phe Ser Glu Asp
           355                 360                 365
   Asn Gly Phe Ser Leu Phe Asp Ala Thr Leu Gln Lys Arg Val Thr Ser
       370                 375                 380
   Asp Glu Arg Ser Asn Phe Gln Glu Ala Cys Asn Asn Ile Leu Asp Ser
   385                 390                 395                 400
   Tyr Glu Met Phe Asn Leu Gln Ser Lys Ala Val Lys Arg Lys Thr Thr
                   405                 410                 415
   Ala Glu Asn Val Asn Thr Gln Ser Ser Arg Asp Ser Glu Ala Thr Arg
               420                 425                 430
   Lys Asn Thr Asn Asp Ala Phe Leu Tyr Ile Tyr Glu Ser Gly Gly Pro
           435                 440                 445
   Gly His Ser Lys Met Thr Glu Pro Ser Leu Gln Asn Lys Asp Ser Ser
       450                 455                 460
   Cys Ser Glu Ser Lys Met Leu Glu Gln Glu Thr Ile Val Ala Ser Glu
   465                 470                 475                 480
   Ala Gly Glu Asn Glu Lys His Lys Lys Ser Phe Leu Glu Arg Ser Ser
                   485                 490                 495
   Leu Glu Asn Pro Cys Gly Thr Ser Leu Glu Met Phe Leu Ser Pro Phe
               500                 505                 510
   Gln Thr Pro Cys His Phe Glu Glu Ser Gly Gln Asp Leu Glu Ile Trp
           515                 520                 525
   Lys Glu Ser Thr Thr Val Asn Gly Met Ala Ala Asn Ile Leu Lys Asn
       530                 535                 540
   Asn Arg Ile Gln Asn Gln Pro Lys Arg Phe Lys Asp Ala Thr Glu Val
   545                 550                 555                 560
   Gly Cys Gln Pro Leu Pro Phe Ala Thr Thr Leu Trp Gly Val His Ser
                   565                 570                 575
   Ala Gln Thr Glu Lys Glu Lys Lys Lys Glu Ser Ser Asn Cys Gly Arg
               580                 585                 590
   Arg Asn Val Phe Ser Tyr Gly Arg Val Lys Leu Cys Ser Thr Gly Phe
           595                 600                 605
   Ile Thr His Val Val Gln Asn Glu Lys Thr Lys Ser Thr Glu Thr Glu
   610 615 620
   His Ser Phe Lys Asn Tyr Val Arg Pro Gly Pro Thr Arg Ala Gln Glu
   625 630 635 640
   Thr Phe Gly Asn Arg Thr Arg His Ser Val Glu Thr Pro Asp Ile Lys
   645 650 655
   Asp Leu Ala Ser Thr Leu Ser Lys Glu Ser Gly Gln Leu Pro Asn Lys
   660 665 670
   Lys Asn Cys Arg Thr Asn Ile Ser Tyr Gly Leu Glu Asn Glu Pro Thr
   675 680 685
   Ala Thr Tyr Thr Met Phe Ser Ala Phe Gln Glu Gly Ser Lys Lys Ser
   690 695 700
   Gln Thr Asp Cys Ile Leu Ser Asp Thr Ser Pro Ser Phe Pro Trp Tyr
   705 710 715 720
   Arg His Val Ser Asn Asp Ser Arg Lys Thr Asp Lys Leu Ile Gly Phe
   725 730 735
   Ser Lys Pro Ile Val Arg Lys Lys Leu Ser Leu Ser Ser Gln Leu Gly
   740 745 750
   Ser Leu Glu Lys Phe Lys Arg Gln Tyr Gly Lys Val Glu Asn Pro Leu
   755 760 765
   Asp Thr Glu Val Glu Glu Ser Asn Gly Val Thr Thr Asn Leu Ser Leu
   770 775 780
   Gln Val Glu Pro Asp Ile Leu Leu Lys Asp Lys Asn Arg Leu Glu Asn
   785 790 795 800
   Ser Asp Val Cys Lys Ile Thr Thr Met Glu His Ser Asp Ser Asp Ser
   805 810 815
   Ser Cys Gln Pro Ala Ser His Ile Leu Asp Ser Glu Lys Phe Pro Phe
   820 825 830
   Ser Lys Asp Glu Asp Cys Leu Glu Gln Gln Met Pro Ser Leu Arg Glu
   835 840 845
   Ser Pro Met Thr Leu Lys Glu Leu Ser Leu Phe Asn Arg Lys Pro Leu
   850 855 860
   Asp Leu Glu Lys Ser Ser Glu Ser Leu Ala Ser Lys Leu Ser Arg Leu
   865                 870                 875                 880
   Lys Gly Ser Glu Arg Glu Thr Gln Thr Met Gly Met Met Ser Arg Phe
                   885                 890                 895
   Asn Glu Leu Pro Asn Ser Asp Ser Ser Arg Lys Asp Ser Lys Leu Cys
               900                 905                 910
   Ser Val Leu Thr Gln Asp Phe Cys Met Leu Phe Asn Asn Lys His Glu
           915                 920                 925
   Lys Thr Glu Asn Gly Val Ile Pro Thr Ser Asp Ser Ala Thr Gln Asp
       930                 935                 940
   Asn Ser Phe Asn Lys Asn Ser Lys Thr His Ser Asn Ser Asn Thr Thr
   945                 950                 955                 960
   Glu Asn Cys Val Ile Ser Glu Thr Pro Leu Val Leu Pro Tyr Asn Asn
                   965                 970                 975
   Ser Lys Val Thr Gly Lys Asp Ser Asp Val Leu Ile Arg Ala Ser Glu
               980                 985                 990
   Gln Gln Ile Gly Ser Leu Asp Ser  Pro Ser Gly Met Leu  Met Asn Pro
           995                 1000                1005
   Val Glu  Asp Ala Thr Gly Asp  Gln Asn Gly Ile Cys  Phe Gln Ser
       1010                 1015                1020
   Glu Glu  Ser Lys Ala Arg Ala  Cys Ser Glu Thr Glu  Glu Ser Asn
       1025                 1030                1035
   Thr Cys  Cys Ser Asp Trp Gln  Arg His Phe Asp Val  Ala Leu Gly
       1040                 1045                1050
   Arg Met  Val Tyr Val Asn Lys  Met Thr Gly Leu Ser  Thr Phe Ile
       1055                 1060                1065
   Ala Pro  Thr Glu Asp Ile Gln  Ala Ala Cys Thr Lys  Asp Leu Thr
       1070                 1075                1080
   Thr Val  Ala Val Asp Val Val  Leu Glu Asn Gly Ser  Gln Tyr Arg
       1085                 1090                1095
   Cys Gln  Pro Phe Arg Ser Asp  Leu Val Leu Pro Phe  Leu Pro Arg
       1100                 1105                1110
   Ala Arg  Ala Glu Arg Thr Val  Met Arg Gln Asp Asn  Arg Asp Thr
   1115 1120 1125
   Val Asp Asp Thr Val Ser Ser Glu Ser Leu Gln Ser Leu Phe Ser
   1130 1135 1140
   Glu Trp Asp Asn Pro Val Phe Ala Arg Tyr Pro Glu Val Ala Val
   1145 1150 1155
   Asp Val Ser Ser Gly Gln Ala Glu Ser Leu Ala Val Lys Ile His
   1160 1165 1170
   Asn Ile Leu Tyr Pro Tyr Arg Phe Thr Lys Gly Met Ile His Ser
   1175 1180 1185
   Met Gln Val Leu Gln Gln Val Asp Asn Lys Phe Ile Ala Cys Leu
   1190 1195 1200
   Met Ser Thr Lys Thr Glu Glu Asn Gly Glu Ala Asp Ser Tyr Glu
   1205 1210 1215
   Lys Gln Gln Ala Gln Gly Ser Gly Arg Lys Lys Leu Leu Ser Ser
   1220 1225 1230
   Thr Leu Ile Pro Pro Leu Glu Ile Thr Val Thr Glu Glu Gln Arg
   1235 1240 1245
   Arg Leu Leu Trp Cys Tyr His Lys Asn Leu Glu Asp Leu Gly Leu
   1250 1255 1260
   Glu Phe Val Phe Pro Asp Thr Ser Asp Ser Leu Val Leu Val Gly
   1265 1270 1275
   Lys Val Pro Leu Cys Phe Val Glu Arg Glu Ala Asn Glu Leu Arg
   1280 1285 1290
   Arg Gly Arg Ser Thr Val Thr Lys Ser Ile Val Glu Glu Phe Ile
   1295 1300 1305
   Arg Glu Gln Leu Glu Leu Leu Gln Thr Thr Gly Gly Ile Gln Gly
   1310 1315 1320
   Thr Leu Pro Leu Thr Val Gln Lys Val Leu Ala Ser Gln Ala Cys
   1325 1330 1335
   His Gly Ala Ile Lys Phe Asn Asp Gly Leu Ser Leu Gln Glu Ser
   1340 1345 1350
   Cys Arg Leu Ile Glu Ala Leu Ser Ser Cys Gln Leu Pro Phe Gln
   1355 1360 1365
   Cys Ala  His Gly Arg Pro Ser  Met Leu Pro Leu Ala  Asp Ile Asp
       1370                 1375                 1380
   His Leu  Glu Gln Glu Lys Gln  Ile Lys Pro Asn Leu  Thr Lys Leu
       1385                 1390                 1395
   Arg Lys  Met Ala Gln Ala Trp  Arg Leu Phe Gly Lys  Ala Glu Cys
       1400                 1405                 1410
   Asp Thr  Arg Gln Ser Leu Gln  Gln Ser Met Pro Pro  Cys Glu Pro
       1415                 1420                 1425
   Pro
<210> 22
   <211> 4895
   <212> DNA
   <213> Homo sapiens
<400> 22
   gtcggcgtcc gaggcggttg gtgtcggaga atttgttaag cgggactcca ggcaattatt 60
   tccagtcaga gaaggaaacc agtgcctggc attctcacca tctttctacc taccatgatc 120
   aagtgcttgt cagttgaagt acaagccaaa ttgcgttctg gtttggccat aagctccttg 180
   ggccaatgtg ttgaggaact tgccctcaac agtattgatg ctgaagcaaa atgtgtggct 240
   gtcagggtga atatggaaac cttccaagtt caagtgatag acaatggatt tgggatgggg 300
   agtgatgatg tagagaaagt gggaaatcgt tatttcacca gtaaatgcca ctcggtacag 360
   gacttggaga atccaaggtt ttatggtttc cgaggagagg ccttggcaaa tattgctgac 420
   atggccagtg ctgtggaaat ttcgtccaag aaaaacagga caatgaaaac ttttgtgaaa 480
   ctgtttcaga gtggaaaagc cctgaaagct tgtgaagctg atgtgactag agcaagcgct 540
   gggactactg taacagtgta taacctattt taccagcttc ctgtaaggag gaaatgcatg 600
   gaccctagac tggagtttga gaaggttagg cagagaatag aagctctctc actcatgcac 660
   ccttccattt ctttctcttt gagaaatgat gtttctggtt ccatggttct tcagctccct 720
   aaaaccaaag acgtatgttc ccgattttgt caaatttatg gattgggaaa gtcccaaaag
   780
   ctaagagaaa taagttttaa atataaagag tttgagctta gtggctatat cagctctgaa
   840
   gcacattaca acaagaatat gcagtttttg tttgtgaaca aaagactagt tttaaggaca
   900
   aagctacata aactcattga ctttttatta aggaaagaaa gtattatatg caagccaaag
   960
   aatggtccca ccagtaggca aatgaattca agtcttcggc accggtctac cccagaactc
   1020
   tatggcatat atgtaattaa tgtgcagtgc caattctgtg agtatgatgt gtgcatggag
   1080
   ccagccaaaa ctctgattga atttcagaac tgggacactc tcttgttttg cattcaggaa
   1140
   ggagtgaaaa tgtttttaaa gcaagaaaaa ttatttgtgg aattatcagg tgaggatatt
   1200
   aaggaattta gtgaagataa tggttttagt ttatttgatg ctactcttca gaagcgtgtg
   1260
   acttccgatg agaggagcaa tttccaggaa gcatgtaata atattttaga ttcctatgag
   1320
   atgtttaatt tgcagtcaaa agctgtgaaa agaaaaacta ctgcagaaaa cgtaaacaca
   1380
   cagagttcta gggattcaga agctaccaga aaaaatacaa atgatgcatt tttgtacatt
   1440
   tatgaatcag gtggtccagg ccatagcaaa atgacagagc catctttaca aaacaaagac
   1500
   agctcttgct cagaatcaaa gatgttagaa caagagacaa ttgtagcatc agaagctggt
   1560
   gaaaatgaga aacataaaaa atctttcctg gaacgtagct ctttagaaaa tccgtgtgga
   1620
   accagtttag aaatgttttt aagccctttt cagacaccat gtcactttga ggagagtggg
   1680
   caggatctag aaatatggaa agaaagtact actgttaatg gcatggctgc caacatcttg
   1740
   aaaaataata gaattcagaa tcaaccaaag agatttaaag atgctactga agtgggatgc
   1800
   cagcctctgc cttttgcaac aacattatgg ggagtacata gtgctcagac agagaaagag
   1860
   aaaaaaaaag aatctagcaa ttgtggaaga agaaatgttt ttagttatgg gcgagttaaa
   1920
   ttatgttcca ctggctttat aactcatgta gtacaaaatg aaaaaactaa atcaactgaa
   1980
   acagaacatt catttaaaaa ttatgttaga cctggtccca cacgtgccca agaaacattt
   2040
   ggaaatagaa cacgtcattc agttgaaact ccagacatca aagatttagc cagcacttta 2100
   agtaaagaat ctggtcaatt gcccaacaaa aaaaattgca gaacgaatat aagttatggg 2160
   ctagagaatg aacctacagc aacttataca atgttttctg cttttcagga aggtagcaaa 2220
   aaatcacaaa cagattgcat attatctgat acatccccct ctttcccctg gtatagacac 2280
   gtttccaatg atagtaggaa aacagataaa ttaattggtt tctccaaacc aatcgtccgt 2340
   aagaagctaa gcttgagttc acagctagga tctttagaga agtttaagag gcaatatggg 2400
   aaggttgaaa atcctctgga tacagaagta gaggaaagta atggagtcac taccaatctc 2460
   agtcttcaag ttgaacctga cattctgctg aaggacaaga accgcttaga gaactctgat 2520
   gtttgtaaaa tcactactat ggagcatagt gattcagata gtagttgtca accagcaagc 2580
   cacatccttg actcagagaa gtttccattc tccaaggatg aagattgttt agaacaacag 2640
   atgcctagtt tgagagaaag tcctatgacc ctgaaggagt tatctctctt taatagaaaa 2700
   cctttggacc ttgagaagtc atctgaatca ctagcctcta aattatccag actgaagggt 2760
   tccgaaagag aaactcaaac aatggggatg atgagtcgtt ttaatgaact tccaaattca 2820
   gattccagta ggaaagacag caagttgtgc agtgtgttaa cacaagattt ttgtatgtta 2880
   tttaacaaca agcatgaaaa aacagagaat ggtgtcatcc caacatcaga ttctgccaca 2940
   caggataatt cctttaataa aaatagtaaa acacattcta acagcaatac aacagagaac 3000
   tgtgtgatat cagaaactcc tttggtattg ccctataata attctaaagt taccggtaaa 3060
   gattcagatg ttcttatcag agcctcagaa caacagatag gaagtcttga ctctcccagt 3120
   ggaatgttaa tgaatccggt agaagatgcc acaggtgacc aaaatggaat ttgttttcag 3180
   agtgaggaat ctaaagcaag agcttgttct gaaactgaag agtcaaacac gtgttgttca 3240
   gattggcagc ggcatttcga tgtagccctg ggaagaatgg tttatgtcaa caaaatgact 3300
   ggactcagca cattcattgc cccaactgag gacattcagg ctgcttgtac taaagacctg 3360
   acaactgtgg ctgtggatgt tgtacttgag aatgggtctc agtacaggtg tcaacctttt 3420
   agaagcgacc ttgttcttcc tttccttccg agagctcgag cagagaggac tgtgatgaga 3480
   caggataaca gagatactgt ggatgatact gttagtagcg aatcgcttca gtctttgttc 3540
   tcagaatggg acaatccagt atttgcccgt tatccagagg ttgctgttga tgtaagcagt 3600
   ggccaggctg agagcttagc agttaaaatt cacaacatct tgtatcccta tcgtttcacc 3660
   aaaggaatga ttcattcaat gcaggttctc cagcaagtag ataacaagtt tattgcctgt 3720
   ttgatgagca ctaagactga agagaatggc gaggcagatt cctacgagaa gcaacaggca 3780
   caaggctctg gtcggaaaaa attactgtct tctactctaa ttcctccgct agagataaca 3840
   gtgacagagg aacaaaggag actcttatgg tgttaccaca aaaatctgga agatctgggc 3900
   cttgaatttg tatttccaga cactagtgat tctctggtcc ttgtgggaaa agtaccacta 3960
   tgttttgtgg aaagagaagc caatgaactt cggagaggaa gatctactgt gaccaagagt 4020
   attgtggagg aatttatccg agaacaactg gagctactcc agaccaccgg aggcatccaa 4080
   gggacattgc cactgactgt ccagaaggtg ttggcatccc aagcctgcca tggggccatt 4140
   aagtttaatg atggcctgag cttacaggaa agttgccgcc ttattgaagc tctgtcctca 4200
   tgccagctgc cattccagtg tgctcacggg agaccttcta tgctgccgtt agctgacata 4260
   gaccacttgg aacaggaaaa acagattaaa cccaacctca ctaaacttcg caaaatggcc 4320
   caggcctggc gtctctttgg aaaagcagag tgtgatacaa ggcagagcct gcagcagtcc 4380
   atgcctccct gtgagccacc atgagaacag aatcactggt ctaaaaggaa caaagggatg 4440
   ttcactgtat gcctctgagc agagagcagc agcagcaggt accagcacgg ccctgactga 4500
   atcagcccag tgtccctgag cagcttagac agcagggctc tctgtatcag tctttcttga 4560
   gcagatgatt cccctagttg agtagccaga tgaaattcaa gcctaaagac aattcattca 4620
   tttgcatcca tgggcacaga aggttgctat atagtatcta ccttttgcta cttatttaat
   4680
   gataaaattt aatgacagtt taaaaaaaaa aaaaaaaaaa attatttgaa ggggtgggtg
   4740
   atttttgttt ttgtacagtt ttttttcaag cttcacattt gcgtgtatct aattcagctg
   4800
   atgctcaagt ccaaggggta gtctgccttc ccaggctgcc cccagggttt ctgcactggt
   4860
   cccctctttt cccttcagtc ttcttcactt ccctt
   4895
<210> 23
   <211> 16
   <212> PRT
   <213> Homo sapiens
<400> 23
   Ala Val Lys Glu Leu Val Glu Asn Ser Leu Asp Ala Gly Ala Thr Asn
   1 5 10 15
<210> 24
   <211> 48
   <212> PRT
   <213> Homo sapiens
<400> 24
   Leu Arg Pro Asn Ala Val Lys Glu Leu Val Glu Asn Ser Leu Asp Ala
   1 5 10 15
   Gly Ala Thr Asn Val Asp Leu Lys Leu Lys Asp Tyr Gly Val Asp Leu
   20 25 30
   Ile Glu Val Ser Gly Asn Gly Cys Gly Val Glu Glu Glu Asn Phe Glu
   35 40 45
<210> 25
   <211> 21
   <212> DNA
   <213> Homo sapiens
<400> 25
   tgactacttt tgacttcagc c
   21
<210> 26
   <211> 22
   <212> DNA
   <213> Homo sapiens
<400> 26
   aaccattcaa catttttaac cc
   22
<210> 27
   <211> 332
   <212> PRT
   <213> Homo sapiens
<400> 27
   Met Pro Ile Gln Val Leu Pro Pro Gln Leu Ala Asn Gln Ile Ala Ala
   1               5                   10                  15
   Gly Glu Val Val Glu Arg Pro Ala Ser Val Val Lys Glu Leu Val Glu
               20                  25                  30
   Asn Ser Leu Asp Ala Gly Ala Thr Arg Ile Asp Ile Asp Ile Glu Arg
           35                  40                  45
   Gly Gly Ala Lys Leu Ile Arg Ile Arg Asp Asn Gly Cys Gly Ile Lys
       50                  55                  60
   Lys Asp Glu Leu Ala Leu Ala Leu Ala Arg His Ala Thr Ser Lys Ile
   65                  70                  75                  80
   Ala Ser Leu Asp Asp Leu Glu Ala Ile Ile Ser Leu Gly Phe Arg Gly
                   85                  90                  95
   Glu Ala Leu Ala Ser Ile Ser Ser Val Ser Arg Leu Thr Leu Thr Ser
               100                 105                 110
   Arg Thr Ala Glu Gln Gln Glu Ala Trp Gln Ala Tyr Ala Glu Gly Arg
           115                 120                 125
   Asp Met Asn Val Thr Val Lys Pro Ala Ala His Pro Val Gly Thr Thr
       130                 135                 140
   Leu Glu Val Leu Asp Leu Phe Tyr Asn Thr Pro Ala Arg Arg Lys Phe
   145                 150                 155                 160
   Leu Arg Thr Glu Lys Thr Glu Phe Asn His Ile Asp Glu Ile Ile Arg
                   165                 170                 175
   Arg Ile Ala Leu Ala Arg Phe Asp Val Thr Ile Asn Leu Ser His Asn
               180                 185                 190
   Gly Lys Ile Val Arg Gln Tyr Arg Ala Val Pro Glu Gly Gly Gln Lys
           195                 200                 205
   Glu Arg Arg Leu Gly Ala Ile Cys Gly Thr Ala Phe Leu Glu Gln Ala
       210                 215                 220
   Leu Ala Ile Glu Trp Gln His Gly Asp Leu Thr Leu Arg Gly Trp Val
   225                 230                 235                 240
   Ala Asp Pro Asn His Thr Thr Pro Ala Leu Ala Glu Ile Gln Tyr Cys
                   245                 250                 255
   Tyr Val Asn Gly Arg Met Met Arg Asp Arg Leu Ile Asn His Ala Ile
               260                 265                 270
   Arg Gln Ala Cys Glu Asp Lys Leu Gly Ala Asp Gln Gln Pro Ala Phe
           275                 280                 285
   Val Leu Tyr Leu Glu Ile Asp Pro His Gln Val Asp Val Asn Val His
       290                 295                 300
   Pro Ala Lys His Glu Val Arg Phe His Gln Ser Arg Leu Val Arg Asp
   305                 310                 315                 320
   Phe Ile Tyr Gln Gly Val Leu Ser Val Leu Gln Gln
                   325                 330